(19) Europäisches Patentamt
European Patent Office
Office européen des brevets

(11) Veröffentlichungsnummer: **0 166 287**
**B1**

(12) # EUROPÄISCHE PATENTSCHRIFT

(45) Veröffentlichungstag der Patentschrift: **23.08.89**

(51) Int. Cl.⁴: **C 07 D 401/12,** C 07 D 491/04, A 61 K 31/44

(21) Anmeldenummer: **85107104.3**

(22) Anmeldetag: **10.06.85**

(54) **Dialkoxypyridine, Verfahren zu ihrer Herstellung, ihre Anwendung und sie enthaltende Arzneimittel.**

(30) Priorität: **16.06.84 CH 2899/84**
**16.06.84 CH 2901/84**

(43) Veröffentlichungstag der Anmeldung: **02.01.86 Patentblatt 86/1**

(45) Bekanntmachung des Hinweises auf die Patenterteilung: **23.08.89 Patentblatt 89/34**

(84) Benannte Vertragsstaaten: **AT BE CH DE FR GB IT LI LU NL SE**

(56) Entgegenhaltungen:
**EP-A-0 005 129**
**EP-A-0 074 341**
**DE-A-3 132 613**

**Die Akte enthält technische Angaben, die nach dem Eingang der Anmeldung eingereicht wurden und die nicht in dieser Patentschrift enthalten sind.**

(73) Patentinhaber: **Byk Gulden Lomberg Chemische Fabrik GmbH, Byk- Gulden- Strasse 2, D-7750 Konstanz (DE)**

(72) Erfinder: **Kohl, Bernhard, Dr., Heinrich- v.- Tettingen Strasse 35a, D-7750 Konstanz 19 (DE)**
Erfinder: **Sturm, Ernst, Dr., In de Reben 1, D-7750 Konstanz 19 (DE)**
Erfinder: **Klemm, Kurt, Prof. Dr., Im Weinberg 2, D-7753 Allensbach (DE)**
Erfinder: **Riedel, Richard, Dr., Salmannsweilergasse 36, D-7750 Konstanz (DE)**
Erfinder: **Figala, Volker Dr., Am Hochfirst 2, D-7753 Allensbach 4 (DE)**
Erfinder: **Rainer, Georg, Dr., Josef- Anton- Feuchtmayer- Strasse 7, D-7750 Konstanz (DE)**
Erfinder: **Schaefer, Hartmann, Dr., Zum Purren 27, D-7750 Konstanz 16 (DE)**
Erfinder: **Senn- Bilfinger, Jörg, Dr., Säntisstrasse 7, D-7750 Konstanz (DE)**

**EP 0 166 287 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

### Anwendungsgebiet der Erfindung

Die Erfindung betrifft neue Dialkoxypyridine, Verfahren zu ihrer Herstellung, ihre Anwendung und sie enthaltende Arzneimittel. Die erfindungsgemäßen Verbindungen werden in der pharmazeutischen Industrie als Zwischenprodukte und zur Herstellung von Medikamenten verwendet.

### Stand der Technik

In der europäischen Patentanmeldung 0 005 129 werden substituierte Pyridylsulfinylbenzimidazole beschrieben, die magensäuresekretionshemmende Eigenschaften besitzen sollen. - In der europäischen Patentanmeldung 0 074 341 wird die Verwendung einer Reihe von Benzimidazolderivaten zur Magensäuresekretionshemmung beschrieben. In der britischen Patentanmeldung GB 2 082 580 werden tricyclische Imidazolderivate beschrieben, die die Magensäuresekretion hemmen und die Entstehung von Ulcera verhindern sollen.

Es wurde nun überraschenderweise gefunden, daß die unten näher beschriebenen Dialkoxypyridine interessante und unerwartete Eigenschaften aufweisen, durch die sie sich in vorteilhafter Weise von den bekannten Verbindungen unterscheiden.

### Beschreibung der Erfindung

Gegenstand der Erfindung sind neue Dialkoxypyridine der allgemeinen Formel I

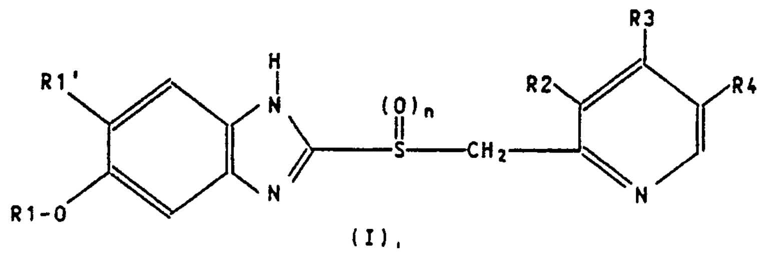

(I),

worin

R1 einen ganz oder überwiegend durch Fluor substituierten 1-3C-Alkylrest oder einen Chlordifluormethylrest und

R1' ein Wasserstoff- oder Halogenatom, Trifluormethyl, einen 1-3C-Alkylrest oder einen gegebenenfalls ganz oder überwiegend durch Fluor substituierten 1-3C-Alkoxyrest, oder

R1 und R1' gemeinsam und unter Einschluß des Sauerstoffatoms, an das R1 gebunden ist, einen gegebenenfalls ganz oder teilweise durch Fluor substituierten 1-2C-Alkylendioxyrest oder einen Chlortrifluorethylendioxyrest darstellen,

R3 einen 1-3C-Alkoxyrest,

einer der Reste R2 und R4 einen 1-3C-Alkoxyrest und der andere ein Wasserstoffatom oder einen 1-3C-Alkylrest und

n die Zahlen 0 oder 1 darstellt,

sowie die Salze dieser Verbindungen.

Als ganz oder überwiegend durch Fluor substituierte 1-3C-Alkylreste seien beispielsweise der 1,1,2-Trifluorethylrest, der Perfluorpropylrest, der Perfluorethylrest und insbesondere der 1,1,2,2-Tetrafluorethylrest, der Trifluormethylrest, der 2,2,2-Trifluorethylrest und der Difluormethylrest genannt.

Halogenatom im Sinne der Erfindung ist ein Brom-, Chlor- und insbesondere ein Fluoratom.

1-3C-Alkylreste sind der Propyl-, Isopropyl-, Ethyl- und insbesondere der Methylrest.

1-3C-Alkoxyreste enthalten neben dem Sauerstoffatom die vorstehend genannten 1-3C-Alkylreste. Bevorzugt ist der Methoxyrest.

Ganz oder überwiegend durch Fluor substituierte 1-3C-Alkoxyreste enthalten neben dem Sauerstoffatom die vorstehend aufgeführten ganz oder überwiegend durch Fluor substituierten 1-3C-Alkylreste. Beispielsweise seien der 1,1,2,2-Tetrafluorethoxy-, der Trifluormethoxy-, der 2,2,2-Trifluorethoxy- und der Difluormethoxyrest genannt.

Als gegebenenfalls ganz oder teilweise durch Fluor substituierte 1-2C-Alkylendioxyreste seien beispielsweise der 1,1-Difluorethylendioxyrest ($-O-CF_2-CH_2-O-$), der 1,1,2,2-Tetrafluorethylendioxyrest ($-O-CF_2-CF_2-O-$), der 1,1,2-Trifluorethylendioxyrest ($-O-CF_2-CHF-O-$) und insbesondere der Difluormethylendioxyrest ($-O-CF_2-O-$) als substituierte, und der Ethylendioxyrest und der Methylendioxyrest als unsubstituierte Reste genannt.

Als Salze kommen für Verbindungen der allgemeinen Formel I, in denen n die Zahl 0 bedeutet (Sulfide), bevorzugt alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Embonat, Metembonat, Stearat, Tosilat, 2-Hydroxy-3-naphthoat, 3-Hydroxy-2-naphthoat oder Mesilat.

Für Verbindungen der allgemeinen Formel I, in denen n die Zahl 1 bedeutet (Sulfoxide), kommen als Salze bevorzugt basische Salze in Betracht, insbesondere pharmakologisch verträgliche Salze mit in der Galenik üblicherweise verwendeten anorganischen und organischen Basen. Als Beispiele für basische Salze seien Natrium-, Kalium-, Calcium- oder Aluminiumsalze erwähnt.

Eine Ausgestaltung (Ausgestaltung a) der Erfindung sind Verbindungen der allgemeinen Formel I, worin R1' ein Wasserstoffatom darstellt und R1, R2, R3, R4 und n die oben angegebenen Bedeutungen haben, und ihre Salze.

Eine weitere Ausgestaltung (Ausgestaltung b) der Erfindung sind Verbindungen der allgemeinen Formel I, worin R1' Halogen, Trifluormethyl, einen 1-3C-Alkylrest oder einen gegebenenfalls ganz oder überwiegend durch Fluor substituierten 1-3C-Alkoxyrest darstellt und R1, R2, R3, R4 und n die oben angegebenen Bedeutungen haben, und ihre Salze.

Eine weitere Ausgestaltung (Ausgestaltung c) der Erfindung sind Verbindungen der allgemeinen Formel I, worin R1 und R1' gemeinsam und unter Einschluß des Sauerstoffatoms, an das R1 gebunden ist, einen 1-2C-Alkylendioxyrest darstellen und R2, R3, R4 und n die oben angegebenen Bedeutungen haben, und ihre Salze.

Eine weitere Ausgestaltung (Ausgestaltung d) der Erfindung sind Verbindungen der allgemeinen Formel I, worin R1 und R1' gemeinsam und unter Einschluß des Sauerstoffatoms, an das R1 gebunden ist, einen ganz oder teilweise durch Fluor substituierten 1-2C-Alkylendioxyrest darstellen und R2, R3, R4 und n die oben angegebenen Bedeutungen haben, und ihre Salze.

Eine weitere Ausgestaltung (Ausgestaltung e) der Erfindung sind Verbindungen der allgemeinen Formel I, worin R1 und R1' gemeinsam und unter Einschluß des Sauerstoffatoms, an das R1 gebunden ist, einen Chlortrifluorethylendioxyrest darstellen und R2, R3, R4 und n die oben angegebenen Bedeutungen haben, und ihre Salze.

Bevorzugte Verbindungen der Ausgestaltung a sind solche der allgemeinen Formel I, worin R1 1,1,2,2-Tetrafluorethyl, Trifluormethyl, 2,2,2-Trifluorethyl, Difluormethyl oder Chlordifluormethyl, R1' ein Wasserstoffatom, R3 Methoxy, einer der Reste R2 und R4 Methoxy und der andere ein Wasserstoffatom oder Methyl und n die Zahlen 0 oder 1 darstellt, und die Salze dieser Verbindungen.

Bevorzugte Verbindungen der Ausgestaltung b sind solche der allgemeinen Formel I, worin R1 Difluormethyl, R1' Difluormethoxy oder Methoxy, R3 Methoxy, einer der Reste R2 und R4 Methoxy und der andere ein Wasserstoffatom oder Methyl und n die Zahlen 0 oder 1 darstellt, und die Salze dieser Verbindungen.

Bevorzugte Verbindungen der Ausgestaltung c sind solche der allgemeinen Formel I, worin R1 und R1' gemeinsam und unter Einschluß des Sauerstoffatoms, an das R1 gebunden ist, einen Methylen- oder Ethylendioxyrest darstellen, R3 Methoxy, einer der Reste R2 und R4 Methoxy und der andere ein Wasserstoffatom oder Methyl und n die Zahlen 0 oder 1 darstellt, und die Salze dieser Verbindungen.

Bevorzugte Verbindungen der Ausgestaltung d sind solche der allgemeinen Formel I, worin R1 und R1' gemeinsam und unter Einschluß des Sauerstoffatoms, an das R1 gebunden ist, einen Difluormethylendioxyrest oder einen 1,1,2-Trifluorethylendioxyrest darstellen, R3 Methoxy, einer der Reste R2 und R4 Methoxy und der andere ein Wasserstoffatom oder Methyl und n die Zahlen 0 oder 1 darstellt, und die Salze dieser Verbindungen.

Bevorzugte Verbindungen der Ausgestaltung e sind solche der allgemeinen Formel I, worin R1 und R1' gemeinsam und unter Einschluß des Sauerstoffatoms, an das R1 gebunden ist, einen Chlortrifluorethylendioxyrest darstellen, R3 Methoxy, einer der Reste R2 und R4 Methoxy und der andere ein Wasserstoffatom oder Methyl und n die Zahlen 0 oder 1 darstellt, und die Salze dieser Verbindungen.

Als erfindungsgemäße Verbindungen seien beispielsweise genannt:

2-[(4,5-Dimethoxy-3-methyl-2-pyridyl)methylsulfinyl]-5-trifluormethoxy-1H-benzimidazol,
2-[(4,5-Dimethoxy-3-methyl-2-pyridyl)methylthio]-5-trifluormethoxy-1H-benzimidazol,
2-[(4,5-Dimethoxy-3-methyl-2-pyridyl)methylsulfinyl]-5-(1,1,2,2-tetrafluorethoxy)1H-benzimidazol,
2-[(4,5-Dimethoxy-3-methyl-2-pyridyl)methylthio]-5-(1,1,2,2-tetrafluorethoxy)1H-benzimidazol,
2-[(4,5-Dimethoxy-3-methyl-2-pyridyl)methylsulfinyl]-5-(2,2,2-trifluorethoxy)1H-benzimidazol,
2-[(4,5-Dimethoxy-3-methyl-2-pyridyl)methylthio]-5-(2,2,2-trifluorethoxy)1H-benzimidazol,
5-Difluormethoxy-2-[(4,5-dimethoxy-3-methyl-2-pyridyl)methylsulfinyl]-1H-benzimidazol,
5-Difluormethoxy-2-[(4,5-dimethoxy-3-methyl-2-pyridyl)methylthio]-1H-benzimidazol,
5-Chlordifluormethoxy-2-[(4,5-dimethoxy-3-methyl-2-pyridyl)methylsulfinyl]-1H-benzimidazol,
5-Chlordifluormethoxy-2-[(4,5-dimethoxy-3-methyl-2-pyridyl)methylthio]-1H-benzimidazol,
5,6-Bis(difluormethoxy)-2-[(4,5-dimethoxy-3-methyl-2-pyridyl)methylsulfinyl]-1H-benzimidazol,
5,6-Bis(difluormethoxy)-2-[(4,5-dimethoxy-3-methyl-2-pyridyl)methylthio]-1H-benzimidazol

5-Difluormethoxy-6-methoxy-2-[(4,5-dimethoxy-3-methyl-2-pyridyl)methylsulfinyl]-1H-benzimidazol,
5-Difluormethoxy-6-methoxy-2-[(4,5-dimethoxy-3-methyl-2-pyridyl)methylthio]-1H-benzimidazol,
2-[(4,5-Dimethoxy-2-pyridyl)methylsulfinyl)-5-trifluormethoxy-1H-benzimidazol,
2-[(4,5-Dimethoxy-2-pyridyl)methylthio]-5-trifluormethoxy-1H-benzimidazol,
2-[(4,5-Dimethoxy-2-pyridyl)methylsulfinyl]-5-(1,1,2,2-tetrafluorethoxy)-1H-benzimidazol,
2-[(4,5-Dimethoxy-2-pyridyl)methylthio]-5-(1,1,2,2-tetrafluorethoxy)-1H-benzimidazol,
2-[(4,5-Dimethoxy-2-pyridyl)methylsulfinyl]-5-(2,2,2-trifluorethoxy)-1H-benzimidazol,
2-[(4,5-Dimethoxy-2-pyridyl)methylthio)-5-(2,2,2-trifluorethoxy)-1H-benzimidazol,
5-Difluormethoxy-2-[(4,5-dimethoxy-2-pyridyl)methylsulfinyl]-1H-benzimidazol,
5-Difluormethoxy-2-[(4,5-dimethoxy-2-pyridyl)methylthio]-1H-benzimidazol,
5-Chlordifluormethoxy-2-[(4,5-dimethoxy-2-pyridyl)methylsulfinyl]-1H-benzimidazol,
5-Chlordifluormethoxy-2-[(4,5-dimethoxy-2-pyridyl)methylthio]-1H-benzimidazol,
5,6-Bis(difluormethoxy)-2-[(4,5-dimethoxy-2-pyridyl)methylsulfinyl]-1H-benzimidazol,
5,6-Bis(difluormethoxy)-2-[(4,5-dimethoxy-2-pyridyl)methylthio]-1H-benzimidazol,
5-Difluormethoxy-6-methoxy-2-[(4,5-dimethoxy-2-pyridyl)methylsulfinyl]-1H-benzimidazol,
5-Difluormethoxy-6-methoxy-2-[(4,5-dimethoxy-2-pyridyl)methylthio]-1H-benzimidazol,
2-[(3,4-Dimethoxy-5-methyl-2-pyridyl)methylsulfinyl]-5-trifluormethoxy-1H-benzimidazol,
2-[(3,4-Dimethoxy-5-methyl-2-pyridyl)methylthio]-5-trifluormethoxy-1H-benzimidazol,
2-[(3,4-Dimethoxy-5-methyl-2-pyridyl)methylsulfinyl]-5-(1,1,2,2-tetrafluorethoxy)-1H-benzimidazol,
2-[(3,4-Dimethoxy-5-methyl-2-pyridyl)methylthio]-5-(1,1,2,2-tetrafluorethoxy)-1H-benzimidazol,
2-[(3,4-Dimethoxy-5-methyl-2-pyridyl)methylsulfinyl]-5-(2,2,2-trifluorethoxy)-1H-benzimidazol,
2-[(3,4-Dimethoxy-5-methyl-2-pyridyl)methylthio]-5-(2,2,2-trifluorethoxy)-1H-benzimidazol,
5-Difluormethoxy-2-[(3,4-dimethoxy-5-methyl-2-pyridyl)methylsulfinyl]-1H-benzimidazol,
5-Difluormethoxy-2-[(3,4-dimethoxy-5-methyl-2-pyridyl)methylthio]-1H-benzimidazol,
5-Chlordifluormethoxy-2-[(3,4-dimethoxy-5-methyl-2-pyridyl)methylsulfinyl]-1H-benzimidazol,
5-Chlordifluormethoxy-2-[(3,4-dimethoxy-5-methyl-2-pyridyl)methylthio]-1H-benzimidazol,
5,6-Bis(difluormethoxy)-2-[(3,4-dimethoxy-5-methyl-2-pyridyl)methylsulfinyl]-1H-benzimidazol,
5,6-Bis(difluormethoxy)-2-[(3,4-dimethoxy-5-methyl-2-pyridyl)methylthio]-1H-benzimidazol
5-Difluormethoxy-6-methoxy-2-[(3,4-dimethoxy-5-methyl-2-pyridyl)methylsulfinyl]-1H-benzimidazol,
5-Difluormethoxy-6-methoxy-2-[(3,4-dimethoxy-5-methyl-2-pyridyl)methylthio]-1H-benzimidazol,
2-[(3,4-Dimethoxy-2-pyridyl)methylsulfinyl]-5-trifluormethoxy-1H-benzimidazol,
2-[(3,4-Dimethoxy-2-pyridyl)methylthio]-5-trifluormethoxy-1H-benzimidazol,
2-[(3,4-Dimethoxy-2-pyridyl)methylsulfinyl]-5-(1,1,2,2-tetrafluorethoxy)-1H-benzimidazol,
2-[(3,4-Dimethoxy-2-pyridyl)methylthio]-5-(1,1,2,2-tetrafluorethoxy)-1H-benzimidazol,
2-[(3,4-Dimethoxy-2-pyridyl)methylsulfinyl]-5-(2,2,2-trifluorethoxy)-1H-benzimidazol,
2-[(3,4-Dimethoxy-2-pyridyl)methylthio]-5-(2,2,2-trifluorethoxy)-1H-benzimidazol,
5-Difluormethoxy-2-[(3,4-dimethoxy-2-pyridyl)methylsulfinyl]-1H-benzimidazol,
5-Difluormethoxy-2-[(3,4-dimethoxy-2-pyridyl)methylthio]-1H-benzimidazol,
5-Chlordifluormethoxy-2-[(3,4-dimethoxy-2-pyridyl)methylsulfinyl]-1H-benzimidazol,
5-Chlordifluormethoxy-2-[(3,4-dimethoxy-2-pyridyl)methylthio]-1H-benzimidazol,
5,6-Bis(difluormethoxy)-2-[(3,4-dimethoxy-2-pyridyl)methylsulfinyl]-1H-benzimidazol,
5,6-Bis(difluormethoxy)-2-[(3,4-dimethoxy-2-pyridyl)methylthio]-1H-benzimidazol,
5-Difluormethoxy-6-methoxy-2-[(3,4-dimethoxy-2-pyridyl)methylsulfinyl]-1H-benzimidazol,
5-Difluormethoxy-6-methoxy-2-[(3,4-dimethoxy-2-pyridyl)methylthio]-1H-benzimidazol,
2,2-Difluor-6-[(4,5-dimethoxy-2-pyridyl)methylsulfinyl]-5H-[1,3]-dioxolo-[4,5-f]benzimidazol,
2,2-Difluor-6-[(4,5-dimethoxy-2-pyridyl)methylthio]-5H-[1,3]-dioxolo-[4,5-f]benzimidazol,
2,2-Difluor-6-[(3-methyl-4,5-dimethoxy-2-pyridyl)methylthio]-5H-[1,3]-dioxolo[4,5-f]benzimidazol,
2,2-Difluor-6-[(3-methyl-4,5-dimethoxy-2-pyridyl)methylsulfinyl]-5H-[1,3]-dioxolo[4,5-f]benzimidazol,
6-[(4,5-Diethoxy-3-methyl-2-pyridyl)methylthio]-2,2-difluor-5H-[1,3]-dioxolo[4,5-f]benzimidazol,
6-[(4,5-Diethoxy-3-methyl-2-pyridyl)methylsulfinyl]-2,2-difluor-5H-[1,3]-dioxolo[4,5-f]benzimidazol,
6,6,7-Trifluor-6,7-dihydro-2-[(4,5-dimethoxy-3-methyl-2-pyridyl)methylthio]-1H-[1,4]-dioxino[2,3-f]benzimidazol,
6,6,7-Trifluor-6,7-dihydro-2-[(4,5-dimethoxy-3-methyl-2-pyridyl)methylsulfinyl]-1H-[1,4]-dioxino[2,3-f]benzimidazol,
6,6,7-Trifluor-6,7-dihydro-2-[(4,5-dimethoxy-2-pyridyl)methylthio]-1H-[1,4]-dioxino[2,3-f]benzimidazol,
6,6,7-Trifluor-6,7-dihydro-2-[(4,5-dimethoxy-2-pyridyl)methylsulfinyl]-1H-[1,4]-dioxino[2,3-f]benzimidazol,
2-[(4,5-Diethoxy-2-pyridyl)methylthio]-6,6,7-trifluor-6,7-dihydro-1H-[1,4]-dioxino[2,3-f]benzimidazol,
2-[(4,5-Diethoxy-2-pyridyl)methylsulfinyl]-6,6,7-trifluor-6,7-dihydro-1H-[1,4]-dioxino[2,3-f]benzimidazol,
2-[(4,5-Diethoxy-3-methyl-2-pyridyl)methylthio]-6,6,7-trifluor-6,7-dihydro-1H-[1,4]-dioxino[2,3-f]benzimidazol,
2-[(4,5-Diethoxy-3-methyl-2-pyridyl)methylsulfinyl]-6,6,7-trifluor-6,7-dihydro-1H-[1,4]-dioxino[2,3-f]benzimidazol,
6,6-Difluor-6,7-dihydro-2-[(4,5-dimethoxy-2-pyridyl)methylthio]-1H-[1,4]-dioxino[2,3-f]benzimidazol,
6,6-Difluor-6,7-dihydro-2-[(4,5-dimethoxy-2-pyridyl)methylsulfinyl]-1H-[1,4]-dioxino[2,3-f]benzimidazol,
6,6-Difluor-6,7-dihydro-2-[(4,5-dimethoxy-3-methyl-2-pyridyl)methylthio]-1H-[1,4]-dioxino[2,3-f]benzimidazol,
6,6-Difluor-6,7-dihydro-2-[(4,5-dimethoxy-3-methyl-2-pyridyl)methylsulfinyl]-1H-[1,4]-dioxino[2,3-f]benzimidazol,
6,6,7,7-Tetrafluor-6,7-dihydro-2-[(4,5-dimethoxy-2-pyridyl)methylthio]-1H-[1,4]-dioxino[2,3-f]benzimidazol,
6,6,7,7-Tetrafluor-6,7-dihydro-2-[(4,5-dimethoxy-2-pyridyl)methylsulfinyl]-1H-[1,4]-dioxino[2,3-f]benzimidazol,
6,6,7,7-Tetrafluor-6,7-dihydro-2-[(4,5-dimethoxy-3-methyl-2-pyridyl)methylthio]-1H-[1,4]-dioxino[2,3-f]benzimidazol,
6,6,7,7-Tetrafluor-6,7-dihydro-2-[(4,5-dimethoxy-3-methyl-2-pyridyl)methylsulfinyl]-1H-[1,4]-dioxino[2,3-f]benzimidazol,
6-Chlor-6,7,7-trifluor-6,7-dihydro-2-[(4,5-dimethoxy-3-methyl-2-pyridyl)-methylsulfinyl]-1H-[1,4]-dioxino[2,3-f]benzimidazol,
6-Chlor-6,7,7-trifluor-6,7-dihydro-2-[(4,5-dimethoxy-3-methyl-2-pyridyl)-methylthio]-1H-[1,4]-dioxino[2,3-f]benzimidazol,
6-Chlor-6,7,7-trifluor-6,7-dihydro-2-[(4,5-dimethoxy-2-pyridyl)methylsulfinyl]-1H-[1,4]-dioxino[2,3-f]benzimidazol,
6-Chlor-6,7,7-trifluor-6,7-dihydro-2-[(4,5-dimethoxy-2-pyridyl)methylthio]-1H-[1,4]-dioxino[2,3-f]benzimidazol,
2,2-Difluor-6-[(3,4-dimethoxy-2-pyridyl)methylsulfinyl]-5H-[1,3]-dioxolo[4,5-f]benzimidazol,
2,2-Difluor-6-[(3,4-dimethoxy-2-pyridyl)methylthio]-5H-[1,3]dioxolo[4,5-f]benzimidazol,
2,2-Difluor-6-[(3,4-dimethoxy-5-methyl-2-pyridyl)methylthio]-5H-[1,3]-dioxolo[4,5-f]benzimidazol,
2,2-Difluor-6-[(3,4-dimethoxy-5-methyl-2-pyridyl)methylsulfinyl]-5H-[1,3]-dioxolo[4,5-f]benzimidazol,
6-[(3,4-Diethoxy-5-methyl-2-pyridyl)methylthio]-2,2-difluor-5H-[1,3]-dioxolo[4,5-f]benzimidazol,
6-[(3,4-Diethoxy-5-methyl-2-pyridyl)methylsulfinyl]-2,2-difluor-5H-[1,3]-dioxolo[4,5-f]benzimidazol,
6,6,7-Trifluor-6,7-dihydro-2-[(3,4-dimethoxy-5-methyl-2-pyridyl)methylthio]-1H-[1,4]-dioxino[2,3-f]benzimidazol,
6,6,7-Trifluor-6,7-dihydro-2-[(3,4-dimethoxy-5-methyl-2-pyridyl)methylsulfinyl]-1H-[1,4]-dioxino[2,3-f]benzimidazol,
6,6,7-Trifluor-6,7-dihydro-2-[(3,4-dimethoxy-2-pyridyl)methylthio]-1H-[1,4]-dioxino[2,3-f]benzimidazol,
6,6,7-Trifluor-6,7-dihydro-2-[(3,4-dimethoxy-2-pyridyl)methylsulfinyl]-1H-[1,4]-dioxino[2,3-f]benzimidazol,
2-[(3,4-Diethoxy-2-pyridyl)methylthio]-6,6,7-trifluor-6,7-dihydro-1H-[1,4]-dioxino[2,3-f]benzimidazol,
2-[(3,4-Diethoxy-2-pyridyl)methylsulfinyl]-6,6,7-trifluor-6,7-dihydro-1H-[1,4]-dioxino[2,3-f]benzimidazol,
2-[(3,4-Diethoxy-5-methyl-2-pyridyl)methylthio]-6,6,7-trifluor-6,7-dihydro-1H-[1,4]-dioxino[2,3-f]benzimidazol,
2-[(3,4-Diethoxy-5-methyl-2-pyridyl)methylsulfinyl]-6,6,7-trifluor-6,7-dihydro-1H-[1,4]-dioxino[2,3-f]benzimidazol,
6,6-Difluor-6,7-dihydro-2-[(3,4-dimethoxy-2-pyridyl)methylthio]-1H-[1,4]-dioxino[2,3-f]benzimidazol,
6,6-Difluor-6,7-dihydro-2-[(3,4-dimethoxy-2-pyridyl)methylsulfinyl]-1H-[1,4]-dioxino[2,3-f]benzimidazol,
6,6-Difluor-6,7-dihydro-2-[(3,4-dimethoxy-5-methyl-2-pyridyl)methylthio]-1H-[1,4]-dioxino[2,3-f]benzimidazol,
6,6-Difluor-6,7-dihydro-2-[(3,4-dimethoxy-5-methyl-2-pyridyl)methylsulfinyl]-1H-[1,4]-dioxino[2,3-f]benzimidazol,
6,6,7,7-Tetrafluor-6,7-dihydro-2-[(3,4-dimethoxy-2-pyridyl)methylthio]-1H-[1,4]-dioxino[2,3-f]benzimidazol,
6,6,7,7-Tetrafluor-6,7-dihydro-2-[(3,4-dimethoxy-2-pyridyl)methylsulfinyl]-1H-[1,4]-dioxino[2,3-f]benzimidazol,
6,6,7,7-Tetrafluor-6,7-dihydro-2-[(3,4-dimethoxy-5-methyl-2-pyridyl)-methylthio]-1H-[1,4]-dioxino[2,3-f]benzimidazol,
6,6,7,7-Tetrafluor-6,7-dihydro-2-[(3,4-dimethoxy-5-methyl-2-pyridyl)-methylsulfinyl]-1H-[1,4]-dioxino[2,3-f]benzimidazol,
6-Chlor-6,7,7-trifluor-6,7-dihydro-2-[(3,4-dimethoxy-5-methyl-2-pyridyl)-methylsulfinyl]-1H-[1,4]-dioxino[2,3-f]benzimidazol,
6-Chlor-6,7,7-trifluor-6,7-dihydro-2-[(3,4-dimethoxy-5-methyl-2-pyridyl)-methylthio]-1H-[1,4]-dioxino[2,3-f]benzimidazol,
6-Chlor-6,7,7-trifluor-6,7-dihydro-2-[(3,4-dimethoxy-2-pyridyl)methylsulfinyl]-1H-[1,4]-dioxino[2,3-f]benzimidazol,
6-Chlor-6,7,7-trifluor-6,7-dihydro-2-[(3,4-dimethoxy-2-pyridyl)methylthio]-1H-[1,4]-dioxino[2,3-f]benzimidazol,
6-[(4,5-Dimethoxy-3-methyl-2-pyridyl)methylthio]-5H-[1,3]-dioxolo[4,5-f]benzimidazol,
6-[(4,5-Dimethoxy-3-methyl-2-pyridyl)methylsulfinyl]-5H-[1,3]-dioxolo[4,5-f]benzimidazol,
6-[(4,5-Dimethoxy-2-pyridyl)methylthio]-5H-[1,3]-dioxolo[4,5-f]benzimidazol,
6-[(4,5-Dimethoxy-2-pyridyl)methylsulfinyl]-5H-[1,3]-dioxolo[4,5-f]benzimidazol,
6-[(3,4-Dimethoxy-2-pyridyl)methylthio]-5H-[1,3]-dioxolo[4,5-f]benzimidazol,
6-[(3,4-Dimethoxy-2-pyridyl)methylsulfinyl]-5H-[1,3]-dioxolo[4,5-f]benzimidazol,
6-[(3,4-Dimethoxy-5-methyl-2-pyridyl)methylthio]-5H-[1,3]-dioxolo[4,5-f]benzimidazol,
6-[(3,4-Dimethoxy-5-methyl-2-pyridyl)methylsulfinyl]-5H-[1,3]-dioxolo[4,5-f]benzimidazol,
6,7-Dihydro-2-[(4,5-dimethoxy-3-methyl-2-pyridyl)methylthio]-1H-[1,4]-dioxino[2,3-f]benzimidazol,
6,7-Dihydro-2-[(4,5-dimethoxy-3-methyl-2-pyridyl)methylsulfinyl]-1H-[1,4]-dioxino[2,3-f]benzimidazol,
6,7-Dihydro-2-[(3,4-dimethoxy-5-methyl-2-pyridyl)methylthio]-1H-[1,4]-dioxino[2,3-f]benzimidazol,
6,7-Dihydro-2-[(3,4-dimethoxy-5-methyl-2-pyridyl)methylsulfinyl]-1H-[1,4]-dioxino[2,3-f]benzimidazol,
6,7-Dihydro-2-[(3,4-dimethoxy-2-pyridyl)methylthio]-1H-[1,4]-dioxino[2,3-f]benzimidazol,
6,7-Dihydro-2-[(4,5-dimethoxy-2-pyridyl)methylsulfinyl]-1H-[1,4]-dioxino[2,3-f]benzimidazol
und die Salze dieser Verbindungen.

Bedingt durch die Tautomerie im Imidazolring ist die 5-Substitution im Benzimidazol mit der 6-Substitution

identisch. Entsprechend ist bei den Verbindungen, in denen R1 und R1′ gemeinsam und unter Einschluß des Sauerstoffatoms, an das R1 gebunden ist, einen substituierten Ethylendioxyrest darstellen, die 6-Position im [1,4]-Dioxino[2,3-f]benzimidazolteil mit der 7-Position identisch.

Ein weiterer Gegenstand der Erfindung ist ein Verfahren zur Herstellung der Dialkoxypyridine der allgemeinen Formel I, worin R1, R1′, R2, R3, R4 und n die oben angegebenen Bedeutungen haben, und ihrer Salze.

Das Verfahren ist dadurch gekennzeichnet, daß man

a) Mercaptobenzimidazole der allgemeinen Formel II mit Picolinderivaten III,

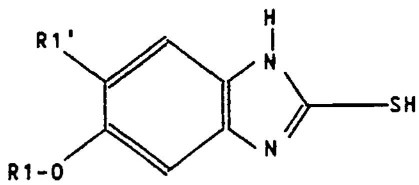

(II)

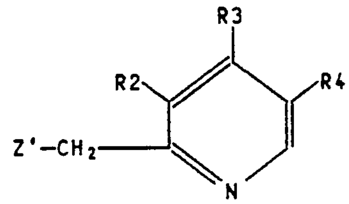

(III),

oder

b) Benzimidazole der allgemeinen Formel IV mit Mercaptopicolinen V,

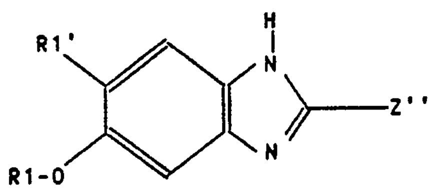

(IV)

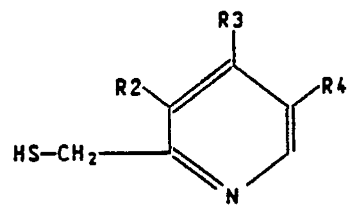

(V),

oder

c) o-Phenylendiamine der allgemeinen Formel VI mit Ameisensäurederivaten VII

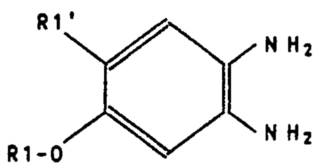

(VI)

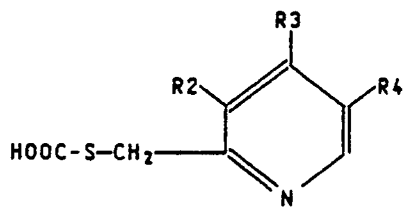

(VII),

umsetzt und gegebenenfalls anschließend die nach a), b) oder c) erhaltenen 2-Benzimidazolyl-2-pyridylmethyl-sulfide der allgemeinen Formel VIII

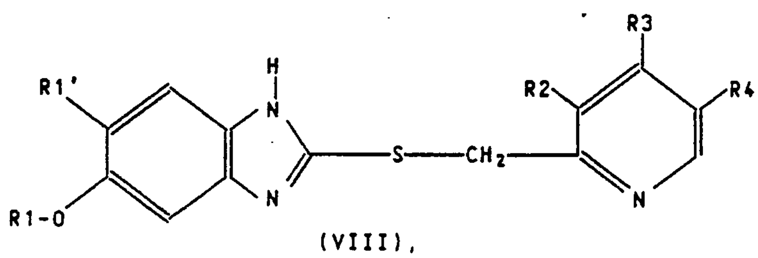

(VIII),

oxidiert und/oder in die Salze überführt,

oder daß man

d) Benzimidazole der allgemeinen Formel IX mit Pyridinderivaten X

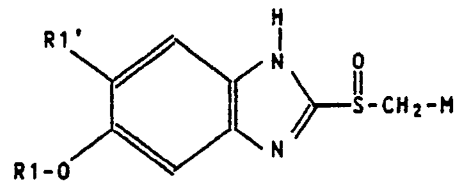

(IX)

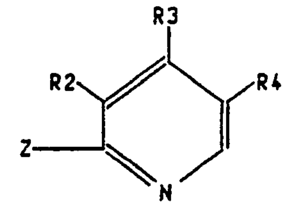

(X),

oder

e) Sulfinylderivate der allgemeinen Formel XI mit 2-Picolinderivaten XII

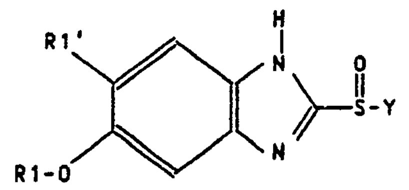

(XI)

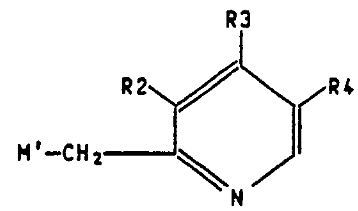

(XII),

umsetzt und gegebenenfalls anschließend in die Salze überführt, wobei

Y, Z, Z' und Z'' geeignete Abgangsgruppen darstellen,

M für ein Alkalimetallatom (Li, Na oder K) steht,

M' für das Äquivalent eines Metallatoms steht und R1, R1', R2, R3, R4 und n die oben angegebenen Bedeutungen haben.

Bei den vorstehend aufgeführten Umsetzungen können die Verbindungen II - XII als solche oder gegebenenfalls in Form ihrer Salze eingesetzt werden.

Die Herstellungsverfahren a), b) und c) führen zu den erfindungsgemäßen Sulfiden, die Oxidation der Verbindungen VIII und die Verfahren d) und e) liefern die erfindungsgemäßen Sulfoxide.

Welche Abgangsgruppen Y, Z, Z' bzw. Z'' geeignet sind, ist dem Fachmann aufgrund seines Fachwissens geläufig. Eine geeignete Abgangsgruppe Y ist beispielsweise eine Gruppe, die zusammen mit der Sulfinylgruppe, an die sie gebunden ist, ein reaktives Sulfinsäurederivat bildet. Als geeignete Abgangsgruppen Y seien beispielsweise Alkoxy-, Dialkylamino- oder Alkylmercaptogruppen genannt. Als geeignete Abgangsgruppen Z, Z' bzw. Z'' seien beispielsweise Halogenatome, insbesondere Chloratome, oder durch Veresterung (z. B. mit p-Toluolsulfonsäure) aktivierte Hydroxylgruppen genannt. Das Äquivalent eines Metallatoms M' ist beispielsweise ein Alkalimetall- (Li, Na oder K), oder ein Erdalkalimetallatom (z. B. Mg), das durch ein Halogenatom (z. B. Br, Grignard-Reagenz) substituiert ist, oder irgendein anderes, gegebenenfalls substituiertes Metallatom, von dem bekannt ist, daß es bei Substitutionsreaktionen metallorganischer Verbindungen wie die obenerwähnten Metalle reagiert.

Die Umsetzung von II mit III erfolgt in an sich bekannter Weise in geeigneten, vorzugsweise polaren protischen oder aprotischen Lösungsmitteln (wie Methanol, Isopropanol, Dimethylsulfoxid, Aceton, Dimethylformamid oder Acetonitril) unter Zusatz oder unter Ausschluß von Wasser. Sie wird beispielsweise in Gegenwart eines Protonenakzeptors durchgeführt. Als solche eignen sich Alkalimetallhydroxide, wie Natriumhydroxid, Alkalimetallcarbonate, wie Kaliumcarbonat, oder tertiäre Amine, wie Pyridin, Triethylamin oder Ethyldiisopropylamin. Alternativ kann die Umsetzung auch ohne Protonenakzeptor durchgeführt werden, wobei - je nach Art der Ausgangsverbindungen - gegebenenfalls zunächst die Säureadditionssalze in besonders reiner Form abgetrennt werden können. Die Reaktionstemperatur kann zwischen 0° und 150° C liegen, wobei in Gegenwart von Protonenakzeptoren Temperaturen zwischen 20° und 80° C und ohne Protonenakzeptoren zwischen 60° und 120° C - insbesondere die Siedetemperatur der verwendeten Lösungsmittel - bevorzugt sind. Die Reaktionszeiten liegen zwischen 0,5 und 24 Stunden.

Bei der Umsetzung von IV mit V, die in an sich bekannter Weise erfolgt, kommen ähnliche Reaktionsbedingungen wie bei der Umsetzung von II mit III zur Anwendung.

Die Reaktion von VI mit VII wird bevorzugt in polaren, gegebenenfalls wasserhaltigen Lösungsmitteln in Gegenwart einer starken Säure, z. B. Salzsäure, insbesondere bei der Siedetemperatur des verwendeten Lösungsmittels durchgeführt.

Die Oxidation der Sulfide VIII erfolgt in an sich bekannter Weise und unter den Bedingungen, wie sie dem Fachmann für die Oxidation von Sulfiden zu Sulfoxiden geläufig sind [siehe hierzu z. B. J. Drabowicz und M. Mikolajczyk, Organic Preparations And Procedures Int. 14(1-2), 45-89 (1982) oder E. Block in S. Patai, The Chemistry of Functional Groups, Supplement E. Part 1, S. 539 - 608, John Wiley and Sons (Interscience Publication), 1980]. Als Oxidationsmittel kommen alle für die Oxidation von Sulfiden zu Sulfoxiden üblicherweise verwendeten Reagenzien in Frage, z. B. Hypohalogenite, insbesondere Peroxysäuren, wie z. B. Peroxyessigsäure, Trifluorperoxyessigsäure, 3,5-Dinitroperoxybenzoesäure, Peroxymaleinsäure oder bevorzugt m-Chlorperoxybenzoesäure.

Die Reaktionstemperatur liegt (je nach Reaktivität des Oxidationsmittels und Verdünnungsgrad) zwischen -70° C und der Siedetemperatur des verwendeten Lösungsmittels, bevorzugt jedoch zwischen -50° und +20° C. Die Oxidation wird zweckmäßigerweise in inerten Lösungsmitteln, z. B. aromatischen oder chlorierten Kohlenwasserstoffen, wie Benzol, Toluol, Dichlormethan oder Chloroform, oder in Estern, wie Essigsäureethylester oder Essigsäureisopropylester, oder in Ethern, wie Dioxan, mit Zusatz von Wasser oder ohne Wasser durchgeführt.

Die Umsetzung von IX mit X erfolgt bevorzugt in inerten Lösungsmitteln, wie sie auch für die Reaktion von Enolationen mit Alkylierungsmitteln üblicherweise verwendet werden. Beispielsweise seien aromatische Lösungsmittel wie Benzol oder Toluol genannt. Die Reaktionstemperatur liegt (je nach Art des Alkalimetallatoms M und der Abgangsgruppe Z) in der Regel zwischen 0° und 120° C, wobei die Siedetemperatur des verwendeten Lösungsmittels bevorzugt ist. Beispielsweise [wenn M Li (Lithium) und Z Cl (Chlor) darstellt und die Umsetzung in Benzol durchgeführt wird] ist die Siedetemperatur von Benzol (80° C) bevorzugt.

Die Verbindungen XI werden mit den Verbindungen XII in an sich bekannter Weise umgesetzt, wie sie dem Fachmann für die Reaktion metallorganischer Verbindungen geläufig ist.

Je nach Art der Ausgangsverbindungen, die gegebenenfalls auch in Form ihrer Salze eingesetzt werden können, und in Abhängigkeit von den Reaktionsbedingungen werden die erfindungsgemäßen Verbindungen zunächst entweder als solche oder in Form ihrer Salze gewonnen.

Im übrigen erhält man die Salze durch Auflösen der freien Verbindungen in einem geeigneten Lösungsmittel, z. B. in einem chlorierten Kohlenwasserstoff, wie Methylenchlorid oder Chloroform, einem niedermolekularen aliphatischen Alkohol (Ethanol, Isopropanol), einem Ether (Diisopropylether), Keton (Aceton) oder Wasser, das die gewünschte Säure bzw. Base enthält, oder dem die gewünschte Säure bzw. Base - gegebenenfalls in der genau berechneten stöchiometrischen Menge anschließend zugegeben wird.

Die Salze werden durch Filtrieren, Umfällen, Ausfällen oder durch Verdampfen des Lösungsmittels gewonnen.

Erhaltene Salze können durch Alkalisieren bzw. Ansäuern, z. B. mit wäßrigem Natriumhydrogencarbonat bzw. mit verdünnter Salzsäure, in die freien Verbindungen umgewandelt werden, welche wiederum in die Salze übergeführt werden können. Auf diese Weise lassen sich die Verbindungen reinigen, oder es lassen sich pharmakologisch nicht verträgliche Salze in pharmakologisch verträgliche Salze umwandeln.

Die erfindungsgemäßen Sulfoxide sind optisch aktive Verbindungen. Die Erfindung umfaßt daher sowohl die Enantiomeren als auch ihre Mischungen und Racemate. Die Enantiomeren können in an sich bekannter Weise (beispielsweise durch Herstellung und Trennung entsprechender diastereoisomerer Verbindungen) separiert werden. Die Enantiomeren können aber auch durch asymmetrische Synthese, beispielsweise durch Reaktion optisch aktiver reiner Verbindungen XI oder diastereoisomer reiner Verbindungen XI mit Verbindungen XII hergestellt werden [siehe hierzu K.K. Andersen, Tetrahedron Lett., 93 (1962)].

Die erfindungsgemäßen Verbindungen werden bevorzugt durch Umsetzung von II mit III und gegebenenfalls anschließende Oxidation des entstandenen Sulfids VIII synthetisiert.

Die Verbindungen der allgemeinen Formel II sind teils bekannt (DE-OS-3 132 613), oder sie können nach bekannten Vorschriften analog hergestellt werden. Verbindungen II erhält man beispielsweise durch Umsetzen von Verbindungen VI mit Kohlendisulfid in Gegenwart von Alkalihydroxiden oder mit Alkali-O-ethyldithiocarbonaten.

Die Verbindungen VI können nach der im folgenden Reaktionsschema A angegebenen allgemeinen Herstellungsmethode synthetisiert werden:

**Reaktionsschema A:**

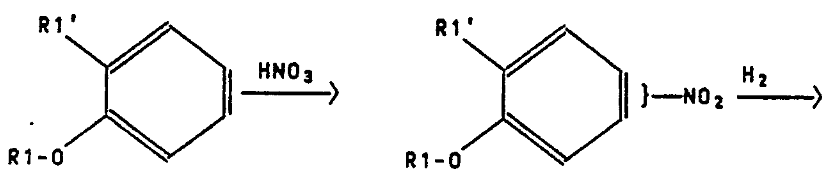


(A1) (A2)

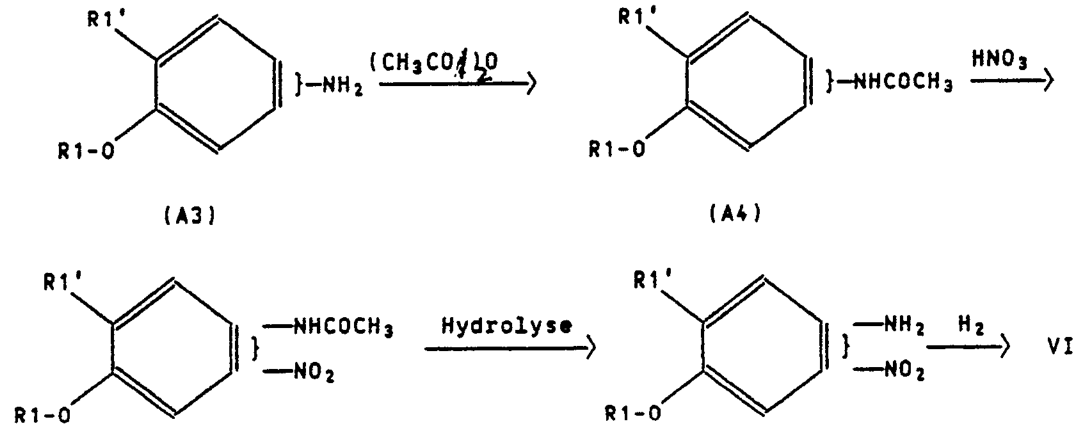


(A3) (A4)

VI

(A5) (A6)

Die Ausgangsverbindungen A1 - A3 können nach bekannten Methoden oder analog zu diesen [z. B. J. Org. Chem. 44, 2907 - 2910 (1979); J. Org. Chem. 29, 1 - 11 (1964); DE-OS-2 029 556; DE-OS-2 848 531; J.Fluorine Chem. 18, 281 - 91 (1981); Synthesis 1980, 727 - 8] hergestellt werden, wobei bei ungleichen Substituenten R1' und R1-O- auch Isomerengemische entstehen können.

Die Verbindungen IV, IX und XI können beispielsweise aus den Verbindungen II in für den Fachmann bekannter Weise hergestellt werden.

Die Verbindungen IX werden beispielsweise aus den Verbindungen II durch Methylierung, Oxydation und anschließende Deprotonierung - z. B. mit Alkalimetallhydriden oder -alkoholaten oder üblichen metallorganischen Verbindungen erhalten. Die Verbindungen X werden in Anlehnung an Z. Talik, Roczniki Chem. 35, 475 (1961) hergestellt.

Die Verbindungen III können - je nach Substitutionsmuster - auf verschiedene Weise hergestellt werden:

1. Verbindungen III mit R2 und R3 = 1-3C-Alkoxy und R4 = Wasserstoffatom oder 1-3C-Alkyl.

Diese Verbindungen werden z. B. ausgehend von bekannten oder auf bekanntem Wege herstellbaren 3-Hydroxy- bzw. 3-Hydroxy-5-alkyl-pyridinen durch Benzylierung der Hydroxygruppe (z. B. mit Kaliumhydroxid und Benzylchlorid in Dimethylsulfoxid), N-Oxidation (z. B. mit 30-%-igem Wasserstoffperoxid), Nitrierung in 4-Position (z. B. mit Nitriersäure), Austausch der Nitrogruppe gegen die 1-3C-Alkoxygruppe (z. B. durch Umsetzung mit Alkali-alkoxid), reduktive Debenzylierung und gleichzeitige N-Deoxygenierung (z. B. mit Wasserstoff an Palladium/Kohle in saurem Medium), Einführung der Hydroxymethylgruppe in o-Position zum Pyridinstickstoff (z. B. durch Umsetzung mit alkalischer Formalinlösung), Umwandlung der 3-Hydroxy- in eine 1-3C-Alkoxygruppe (z. B. durch Alkylierung mit 1-3C-Alkyliodid in basischem Medium) und Einführung der Fluchtgruppe Z' (z. B. durch Umsetzung mit Thionylchlorid) hergestellt. In einer bevorzugten Alternative werden die Verbindungen ausgehend von bekannten oder auf bekanntem Wege herstellbaren 3-Hydroxy-2-alkyl- bzw. 3-Hydroxy-2,5-dialkyl-pyridinen durch Alkylierung der Hydroxygruppe (z. B. mit Kaliumhydroxid und Methyliodid in Dimethylsulfoxid), H-Oxidation (z. B. mit 30-%-igem Wasserstoffperoxid), Nitrierung in 4-Position (z. B. mit Salpetersäure), Austausch der Nitrogruppe gegen die 1-3C-Alkoxygruppe (z. B. durch Umsetzung mit Alkalialkoxid), Umwandlung in das 2-Acetoxymethylpyridin (z. B. mit heißem Essigsäureanhydrid), Verseifung (z. B. mit verdünnter Natronlauge) zur Hydroxymethylgruppe und Einführung der Fluchtgruppe Z' (z. B. durch Umsetzung mit Thionylchlorid) hergestellt.

2. Verbindungen III mit R3 und R4 = 1-3C-Alkoxy und R2 = Wasserstoffatom.

Diese Verbindungen werden z. B. ausgehend vom bekannten 5-Hydroxy-2-methylpyridinen durch Alkylierung der Hydroxygruppe (z. B. mit 1-3C-Alkyliodid und Kaliumhydroxid in Dimethylsulfoxid), N-Oxidation (z. B. mit 30-%-igem Wasserstoffperoxid), Nitrierung in 4-Position (z. B. mit Nitriersäure), Austausch der Nitrogruppe gegen die 1-3C-Alkoxygruppe (z. B. durch Umsetzung mit Alkali-alkoxid), Umwandlung in das 2-Acetoxymethylpyridin (z. B. mit heißem Essigsäureanhydrid), Verseifung (z. B. mit verdünnter Natronlauge) zur 2-Hydroxymethylgruppe und Einführung der Fluchtgruppe Z' (z. B. durch Umsetzung mit Thionylchlorid) hergestellt.

3. Verbindungen III mit R3 und R4 = 1-3C-Alkoxy und R2 = 1-3C-Alkyl.

Diese Verbindungen werden z. B. ausgehend von bekannten oder auf bekanntem Weg herstellbaren 2-Methyl-3-alkyl-4-alkoxypyridinen (siehe z. B. EP-A-0 080 602) durch N-Oxidation (z. B. mit 30-%-igem Wasserstoffperoxid), gezielte Acetoxylierung und anschließende Verseifung in 5-Postition (z. B. mit Essigsäureanhydrid und anschließend Natronlauge), Alkylierung der 5-Hydroxygruppe (z. B. mit 1-3C-Alkyliodid und Natronlauge in Dimethylsulfoxid), N-Oxidation (z. B. mit m-Chlorperoxibenzoesäure), Umwandlung in das 2-Acetoxymethylpyridin (z. B. mit heißem Essigsäureanhydrid), Verseifung (z. B. mit verdünnter Natronlauge) zur

2-Hydroxymethylgruppe und Einführung der Fluchtgruppe Z' (z. B. durch Umsetzung mit Thionylchlorid) hergestellt.

Welche Reaktionsbedingungen (Temperaturen, Reaktionszeiten, Lösungsmittel etc.) bei den oben skizzierten Synthesewegen für die Herstellung der Verbindungen III im einzelnen erforderlich sind, ist dem Fachmann aufgrund seines Fachwissens geläufig. Die genaue Herstellung einzelner Vertreter der Verbindungen III ist in den Beispielen angegeben. Die Herstellung weiterer Vertreter erfolgt in analoger Weise.

Verbindungen der allgemeinen Formel III, worin R3 einen 1-3C-Alkoxyrest darstellt, einer der Reste R2 und R4 einen 1-3C-Alkoxyrest und der andere einen 1-3C-Alkylrest darstellt sind neu und ebenfalls Gegenstand der Erfindung.

Die Verbindungen V, VII und XII werden beispielsweise ausgehend von den Verbindungen III auf für den Fachmann bekannten Wegen hergestellt.

Die folgenden Beispiele erläutern die Erfindung näher, ohne sie einzuschränken. Die in den Beispielen namentlich aufgeführten Verbindungen der Formel I sowie Salze dieser Verbindungen sind bevorzugter Gegenstand der Erfindung. In den Beispielen bedeutet F. Schmelzpunkt, Zers. steht für Zersetzung, Sdp. steht für Siedepunkt.

## Beispiele

1. <u>2-[(4,5-Dimethoxy-2-pyridyl)methylthio]-5-trifluormethoxy-1H-benzimidazol</u>

Zu einer Lösung von 1,64 g 2-Mercapto-5-trifluormethoxy-1H-benzimidazol in 40 ml Ethanol und 20 ml 1 n Natronlauge werden 1,57 g 2-Chlormethyl-4,5-dimethoxypyridiniumchlorid zugegeben, 2 Stunden bei 20°C und anschließend noch 1 Stunde bei 40°C gerührt, Ethanol am Rotationsverdampfer (1 kPa/40°C) abdestilliert, der dabei ausfallende farblose Niederschlag über eine Nutsche filtriert, mit 1 n Natronlauge und Wasser nachgewaschen und getrocknet. Man erhält 2,15 g (79 % d.Th.) der Titelverbindung vom F. 92 - 93°C.

Analog erhält man
5-Chlordifluormethoxy-2-[(4,5-dimethoxy-2-pyridyl)methylthio]-1H-benzimidazol,
5-Difluormethoxy-2-[(4,5-dimethoxy-2-pyridyl)methylthio]-1H-benzimidazol (Öl),
5,6-Bis(difluormethoxy)-2-[(4,5-dimethoxy-2-pyridyl)methylthio]-1H-benzimidazol,
5-Difluormethoxy-6-methoxy-2-[(4,5-dimethoxy-2-pyridyl)methylthio]-1H-benzimidazol (F. 159 - 160°C) und
5-Difluormethoxy-6-fluor-2-[(4,5-dimethoxy-2-pyridyl)methylthio]-1H-benzimidazol
durch Umsetzung von
5-Chlordifluormethoxy-2-mercapto-1H-benzimidazol,
5-Difluormethoxy-2-mercapto-1H-benzimidazol,
5,6-Bis(difluormethoxy)-2-mercapto-1H-benzimidazol,
5-Difluormethoxy-2-mercapto-6-methoxy-1H-benzimidazol und
5-Difluormethoxy-6-fluor-2-mercapto-1H-benzimidazol
mit
2-Chlormethyl-4,5-dimethoxypyridiniumchlorid.

2. <u>2-[(4,5-Dimethoxy-2-pyridyl)methylsulfinyl]-5-trifluormethoxy-1H-benzimidazol</u>

Zu einer Lösung von 0,36 g 2-[4,5-Dimethoxy-2-pyridyl)methylthio]-5-trifluormethoxy-1H-benzimidazol in 10 ml Methylenchlorid tropft man bei -50°C 5,5 ml einer 0,2 m Lösung von m-Chlorperoxybenzoesäure in Methylenchlorid zu und rührt weitere 30 Minuten bei der angegebenen Temperatur. Nach Zugabe von 0,3 ml Triethylamin wird die kalte Reaktionsmischung in 10 ml 5-%-ige Natriumthiosulfat- und 10 ml 5-%-ige Natriumcarbonatlösung eingerührt, nach Phasentrennung wird noch dreimal mit 10 ml Methylenchlorid extrahiert, die vereinigten organischen Phasen werden einmal mit 5 ml einer 5-%-igen Natriumthiosulfatlösung gewaschen, getrocknet, vom Trockenmittel (Magnesiumsulfat) filtriert und eingeengt. Der Rückstand wird mit Diisopropylether zur Kristallisation gebracht und anschließend aus Methylenchlorid/Diisopropylether umgefällt. Man erhält 0,27 g (72 % d.Th.) der Titelverbindung als farblosen Feststoff vom F. 159 - 61°C (Zers.).

Analog erhält man
5-Chlordifluormethoxy-2-[(4,5-dimethoxy-2-pyridyl)methylsulfinyl]-1H-benzimidazol,
5-Difluormethoxy-2-[(4,5-dimethoxy-2-pyridyl)methylsulfinyl]-1H-benzimidazol [F. 159°C (Zers.)],
5,6-Bis(difluormethoxy)-2-[(4,5-dimethoxy-2-pyridyl)methylsulfinyl]-1H-benzimidazol,
5-Difluormethoxy-6-methoxy-2-[(4,5-dimethoxy-2-pyridyl)methylsulfinyl]-1H-benzimidazol und
5-Difluormethoxy-6-fluor-2,2-[(4,5-dimethoxy-2-pyridyl)methylsulfinyl]-1H-benzimidazol
durch Oxidation der weiteren Sulfide des Beispiels 1 mit m-Chlorperoxibenzoesäure.

3. 2-[4,5-Dimethoxy-2-pyridyl)methylthio]-5-(1,1,2,2,-tetrafluorethoxy)-1H-benzimidazol

Nach der in Beispiel 1 angegebenen Arbeitsweise erhält man durch Umsetzung von 1,07 g 2-Mercapto-5-(1,1,2,2-tetrafluorethoxy)-1H-benzimidazol mit 0,90 g 2-Chlormethyl-4,5-dimethoxypyridiniumchlorid in 15 ml Ethanol unter Zusatz von 17 ml 0,5 n Natronlauge 1,40 g der Titelverbindung als gelbes Öl. Umkristallisation aus Petrolether liefert das Produkt in Form farbloser Kristalle vom F. 125 - 127°C. Ausbeute: 1,20 g (72 % d.Th).

4. 2-[(4,5-Dimethoxy-2-pyridyl)methylsulfinyl]-5-(1,1,2,2,-tetrafluorethoxy)-1H-benzimidazol

Nach der in Beispiel 2 angegebenen Arbeitsweise erhält man durch Oxidation von 0,76 g 2-[(4,5-Dimethoxy-2-pyridyl)methylthio]-5-(1,1,2,2-tetrafluorethoxy)-1H-benzimidazol mit 19 ml einer 0,1 m Lösung von m-Chlorperoxybenzoesäure in 30 ml Methylenchlorid bei -40°C nach Extraktion eine Lösung des Produktes in Methylenchlorid. Nach Trocknung über Magnesiumsulfat wird vom Trockenmittel filtriert, eingeengt und der Rückstand aus Methylenchlorid/Diisopropylether kristallisiert. Man erhält 0,64 g (82 % d.Th.) der Titelverbindung in Form farbloser Kristalle vom F. 160 - 162°C (Zers.).

5. 2-[(4,5-Dimethoxy-2-pyridyl)methylthio]-5-(2,2,2-trifluorethoxy)-1H-benzimidazol

1,0 g 2-Mercapto-5-(2,2,2-trifluorethoxy)-1H-benzimidazol werden in 15 ml Ethanol und 8,5 ml 1 n Natronlauge gelöst, mit 0,90 g 2-Chlormethyl-4,5-dimethoxypyridiniumchlorid versetzt und 20 Stunden gerührt. Nach Zugabe von 30 ml Wasser extrahiert man dreimal mit je 30 ml Methylenchlorid, wäscht die Methylenchloridphase einmal mit 5 ml 0,1 n Natronlauge, trocknet die vereinigten organischen Phasen über Magnesiumsulfat und engt nach Filtration des Trockenmittels vollständig ein. Man erhält 1,51 g (94 % d.Th.) der Titelverbindung als amorphen, festen Rückstand vom F. 55 - 57°C.

6. 2-[(4,5-Dimethoxy-3-pyridyl)methylsulfinyl]-5-(2,2,2-trifluorethoxy)-1H-benzimidazol

0,8 g 2-[(4,5-Dimethoxy-2-pyridyl)methylthio]-5-(2,2,2-trifluorethoxy)-1H-benzimidazol werden in 15 ml Dioxan und 2,5 ml 1 n Natronlauge gelöst. Innerhalb von 2 Stunden wird ein Gemisch von 3 ml 8-prozentiger Natriumhypochloritlösung und 3,5 ml 1 n Natronlauge unter Kühlung auf 0 - 5°C zugetropft. Nach Zugabe von 5 ml 5-%-iger Natriumthiosulfatlösung wird zur Trockene eingeengt, der Rückstand in Wasser aufgenommen und mit Phosphatpuffer auf pH 7 gestellt. Man filtriert vom ausgefallenen Feststoff, trocknet und kristallisiert aus Essigester/Diisopropylether um. Man erhält 0,45 g (55 % d.Th.) der Titelverbindung als farblose Kristalle vom F. 142 - 143°C (Zers.).

7. 2-[(4,5-Dimethoxy-3-methyl-2-pyridyl)methylthio]-5-(1,1,2,2-tetrafluorethoxy)-1H-benzimidazol

Nach der in Beispiel 1 angegebenen Arbeitsweise erhält man durch Umsetzung von 1,07 g 2-Mercapto-5-(1,1,2,2-tetrafluorethoxy)-1H-benzimidazol mit 0,96 g 2-Chlormethyl-4,5-dimethoxy-3-methylpyridiniumchlorid in 12 ml Ethanol unter Zusatz von 17 ml 0,5 n Natronlauge 1,46 g (83 % d.Th.) der Titelverbindung vom F. 127 - 128°C (farbloses Pulver).

8. 2-[(4,5-Dimethoxy-3-methyl-2-pyridyl)methylsulfinyl]-5-(1,1,2,2-tetrafluorethoxy)-1H-benzimidazol

Nach der in Beispiel 2 angegebenen Arbeitsweise erhält man durch Oxidation von 0,99 g 2-[(4,5-Dimethoxy-3-methyl-2-pyridyl)methylthio]-5-(1,1,2,2-tetrafluorethoxy)-1H-benzimidazol mit 12 ml einer 0,2 m Lösung von m-Chlorperoxybenzoesäure in Methylenchlorid bei -40°C und einer Reaktionszeit von 1,5 Stunden 0,8 g eines blaßgelben Öls. Zweimalige Umkristallisation aus Methylenchlorid/Diisopropylether liefert 0,30 g (34 % d.Th.) der Titelverbindung in Form farbloser Kristalle vom F. 125°C (Zers.).

9. 5-Difluormethoxy-2-[(4,5-dimethoxy-3-methyl-2-pyridyl)methylthio)-1H-benzimidazol

Nach der in Beispiel 1 angegebenen Arbeitsweise erhält man durch Umsetzung von 0,38 g (2 mMol) 5-Difluormethoxy-2-mercapto-1H-benzimidazol mit 0,48 g (2 mMol) 2-Chlormethyl-4,5-dimethoxy-3-methylpyridiniumchlorid in 10 ml Ethanol unter Zusatz von 8,8 ml 1 n Natronlauge nach zwei Stunden bei 50°C 0,64 g (84 % d.Th.) der Titelverbindung vom F. 100 - 102°C (farbloses Kristallpulver).

10. 2-[(3,4-Dimethoxy-2-pyridyl)methylthio]-5-(1,1,2,2,-tetrafluorethoxy)-1H-benzimidazol

Zu einer Lösung von 0,46 g (1,7 mMol) 2-Mercapto-5-(1,1,2,2-tetrafluorethoxy)-1H-benzimidazol in 10 ml Ethanol, 10 ml Wasser und 1,8 ml 2 n Natronlauge werden 0,38 g (1,7 mMol) 2-Chlormethyl-3,4-dimethoxypyridiniumchlorid zugegeben; nach einer Stunde Rühren bei 20°C werden erneut 10 ml Wasser zugetropft; anschließend wird bei 20°C nochmals vier Stunden gerührt. Man filtriert vom ausgefallenen Feststoff, wäscht mit 0,01 n Natronlauge und anschließend mit Wasser neutral und trocknet bis zur Gewichtskonstanz. Man erhält 0,63 g (90 % d.Th.) der Titelverbindung als farbloses Kristallpulver vom F. 98 - 102°C.

Analog erhält man
5-Difluormethoxy-2-[(3,4-dimethoxy-2-pyridyl)methylthio]-1H-benzimidazol (F. 104 - 108°C) und
5-Difluormethoxy-6-methoxy-2-[(3,4-dimethoxy-2-pyridyl)methylthio]-1H-benzimidazol (F. 137 - 138°C)
durch Umsetzung von
5-Difluormethoxy-2-mercapto-1H-benzimidazol und
5-Difluormethoxy-6-methoxy-2-mercapto-1H-benzimidazol mit
2-Chlormethyl-3,4-dimethoxypyridiniumchlorid.

11. <u>2-[(4,5-Dimethoxy-3-methyl-2-pyridyl)methylthio]-5-trifluormethoxy-1H-benzimidazol</u>

Nach der in Beispiel 1 angegebenen Arbeitsweise erhält man durch Umsetzung von 1,15 g 2-Mercapto-5-trifluormethoxy-1H-benzimidazol mit 1,20 g 2-Chlormethyl-4,5-dimethoxy-3-methylpyridiniumchlorid in 20 ml Isopropanol unter Zusatz von 20,5 ml 0,5 n Natronlauge 1,40 g (70 % d.Th.) der Titelverbindung. Umkristallisation aus Diisopropylether/Petrolether liefert ein Produkt vom F. 94 - 97°C.

Analog erhält man
2-[(4,5-Dimethoxy-3-methyl-2-pyridyl)methylthio]-5-(2,2,2-trifluorethoxy)-1H-benzimidazol,
5-Chlordifluormethoxy-2-[(4,5-dimethoxy-3-methyl-2-pyridyl)methylthio]-1H-benzimidazol,
5,6-Bis(difluormethoxy)-2-[(4,5-dimethoxy-3-methyl-2-pyridyl)methylthio]-1H-benzimidazol,
5-Difluormethoxy-6-methoxy-2-[(4,5-dimethoxy-3-methyl-2-pyridyl)methylthio]-1H-benzimidazol und
5-Difluormethoxy-6-fluor-2-[(4,5-dimethoxy-3-methyl-2-pyridyl)methylthio]-1H-benzimidazol
durch Umsetzung von
2-Mercapto-5-(2,2,2-trifluorethoxy)-1H-benzimidazol,
5-Chlordifluormethoxy-2-mercapto-1H-benzimidazol,
5,6-Bis(difluormethoxy)-2-mercapto-1H-benzimidazol,
5-Difluormethoxy-2-mercapto-6-methoxy-1H-benzimidazol und
5-Difluormethoxy-6-fluor-2-mercapto-1H-benzimidazol
mit
2-Chlormethyl-4,5-dimethoxy-3-methyl-pyridiniumchlorid.

12. <u>2-[(4,5-Dimethoxy-3-methyl-2-pyridyl)methylsulfinyl]-5-trifluormethoxy-1H-benzimidazol</u>

Nach der in Beispiel 2 angegebenen Arbeitsweise erhält man durch Oxidation von 0,24 g 2-[(4,5-Dimethoxy-3-methyl-2-pyridyl)methylthio]-5-trifluormethoxy-1H-benzimidazol mit 3,3 ml einer 0,2 m Lösung von m-Chlorperoxybenzoesäure in Methylenchlorid bei -50°C und Umfällung aus Methylenchlorid/Diisopropylether 0,19 g (76 % d.Th.) der Titelverbindung als farbloses Pulver; 158 - 159°C Zers.

Analog erhält man
2-[(4,5-Dimethoxy-3-methyl-2-pyridyl)methylsulfinyl]-5-(2,2,2-trifluorethoxy)-1H-benzimidazol,
5-Chlordifluormethoxy-2-[(4,5-dimethoxy-3-methyl-2-pyridyl)methylsulfinyl]-1H-benzimidazol,
5-Difluormethoxy-2-[(4,5-dimethoxy-3-methyl-2-pyridyl)methylsulfinyl]-1H-benzimidazol [F. 133 - 135 (Zers.)],
5,6-Bis(difluormethoxy)-2-[(4,5-dimethoxy-3-methyl-2-pyridyl)methylsulfinyl]-1H-benzimidazol,
5-Difluormethoxy-6-methoxy-2-[(4,5-dimethoxy-3-methyl-2-pyridyl)methylsulfinyl]-1H-benzimidazol,
5-Difluormethoxy-6-fluor-2-[(4,5-dimethoxy-3-methyl-2-pyridyl)methylsulfinyl]1H-benzimidazol
2-[(3,4-Dimethoxy-2-pyridyl)methylsulfinyl]-5-(1,1,2,2-tetrafluorethoxy)-1H-benzimidazol [F. 117 - 119°C (Zers.)] und
5-Difluormethoxy-2-[(3,4-dimethoxy-2-pyridyl)methylsulfinyl]-1H-benzimidazol [F. 136°C (Zers.)]
durch Oxidation der Sulfide der obigen Beispiele 9 bis 11 mit m-Chlorperoxybenzoesäure.

13. <u>2,2-Difluor-6-[(4,5-dimethoxy-3-methyl-2-pyridyl)methylthio]-5H-[1,3]-dioxolo[4,5-f]benzimidazol</u>

Zu einer Lösung von 0,92 g 2,2-Difluor-5H-[1,3]-dioxolo[4,5-f]benzimidazol-6-thiol in 10 ml Ethanol und 10 ml 1 n Natronlauge werden 0,96 g 2-Chlormethyl-4,5-dimethoxy-3-methylpyridiniumchlorid zugegeben. Man rührt die gelbe Reaktionsmischung 1 Stunde bei 20°C, setzt nochmals 10 ml Wasser zu, wobei ein farbloser Feststoff ausfällt, rührt weitere 5 Stunden, filtriert, wäscht mit 1 n Natronlauge und Wasser nach und trocknet bis zur Gewichtskonstanz. Das amorphe Pulver wird aus Methylenchlorid/Diisopropylether umkristallisiert. Man erhält 1,5 g (93 % d.Th.) der Titelverbindung in Form farbloser Kristalle vom F. 160 - 61°C.

Analog erhält man
6,6,7-Trifluor-6,7-dihydro-2-[(4,5-dimethoxy-3-methyl-2-pyridyl)methylthio]-1H-[1,4]-dioxino[2,3-f]benzimidazol,
6-Chlor-6,7,7-trifluor-6,7-dihydro-2-[(4,5-dimethoxy-3-methyl-2-pyridyl)methylthio]-1H-[1,4]-dioxino[2,3-f]benzimidazol und
6,7-Dihydro-2-[(4,5-dimethoxy-3-methyl-2-pyridyl)methylthio]-1H-[1,4]-dioxino[2,3-f]benzimidazol
durch Umsetzung von

6,6,7-Trifluor-6,7-dihydro-1H-[1,4]-dioxino[2,3-f]benzimidazol-2-thiol,
6-Chlor-6,7,7-trifluor-6,7-dihydro-1H-[1,4]-dioxino[2,3-f]benzimidazol-2-thiol bzw.
6,7-Dihydro-1H-[1,4]-dioxino[2,3-f]benzimidazol-2-thiol mit
2-Chlormethyl-4,5-dimethoxy-3-methylpyridiniumchlorid.

14. 2,2-Difluor-6-[(4,5-dimethoxy-3-methyl-2-pyridyl)methylsulfinyl]-5H-[1,3]-dioxolo[4,5-f]benzimidazol

Zu einer auf -40°C gekühlten Suspension von 0,80 g 2,2-Difluor-6-[(4,5-dimethoxy-3-methyl-2-pyridyl)methylthio]-5H-[1,3]-dioxolo[4,5-f]benzimidazol in 10 ml Methylenchlorid tropft man innerhalb von 10 Minuten 21 ml einer 0,1 n Lösung von m-Chlorperoxybenzoesäure in Methylenchlorid zu. Man rührt weitere 20 Minuten und läßt die Temperatur dabei auf -20°C ansteigen, setzt 0,5 ml Triethylamin zu und gießt das Reaktionsgemisch in 40 ml einer jeweils 5-%-igen Natriumthiosulfat- und 5-%-igen Natriumcarbonatlösung ein. Nach Phasentrennung wird die Wasserphase noch zweimal mit je 20 ml Methylenchlorid extrahiert; die vereinigten organischen Phasen werden mit einem Gemisch aus jeweils 5 ml Natriumthiosulfat- und Natriumcarbonatlösung gewaschen, getrocknet und eingeengt. Der Rückstand wird aus Methylenchlorid/Diisopropylether umkristallisiert. Man erhält 0,62 g (75 % d.Th.) der Titelverbindung: Zers. 189 - 90°C.

Analog erhält man
6,6,7-Trifluor-6,7-dihydro-2-[(4,5-dimethoxy-3-methyl-2-pyridyl)methylsulfinyl]-1H-[1,4]-dioxino[2,3-f]benzimidazol,
6-Chlor-6,7,7-trifluor-6,7-dihydro-2-[(4,5-dimethoxy-3-methyl-2-pyridyl)methylsulfinyl]-1H-[1,4]-dioxino[2,3-f]benzimidazol und
6,7-Dihydro-2-[(4,5-dimethoxy-3-methyl-2-pyridyl)methylsulfinyl]-1H-[1,4]-dioxino[2,3-f]benzimidazol
durch Oxidation der unter Beispiel 13 genannten weiteren Sulfide mit m-Chlorperoxybenzoesäure.

15. 6-[(4,5-Dimethoxy-2-pyridyl)methylthio]-5H-[1,3]-dioxolo[4,5-f]benzimidazol

Nach der in Beispiel 13 beschriebenen Arbeitsweise erhält man durch Umsetzung von 0,85 g 5H-[1,3]-dioxolo[4,5-f]benzimidazol-6-thiol mit 0,98 g 2-Chlormethyl-4,5-dimethoxypyridiniumchlorid in 10 ml Ethanol und 10 ml Wasser unter Zusatz von 8,5 ml 1 n Natronlauge nach einer Reaktionszeit von 20 Stunden und nach Einengen des Lösungsmittels im Vakuum auf ein Volumen von 10 ml einen bräunlichen Feststoff. Man löst das Rohprodukt in 30 ml Methylenchlorid, klärt mit Bleicherde (z. B. Tonsil®), engt ein, kristallisiert durch Zugabe von Diisopropylether und kocht den nun blaßgelben Feststoff in 5 ml Methanol aus. Man erhält 0,90 g (60 % d.Th.) der Titelverbindung als farblosen Feststoff vom F. 198 - 200°C.

16. 6-[(4,5-Dimethoxy-2-pyridyl)methylsulfinyl]-5H-[1,3]-dioxolo[4,5-f]benzimidazol

Nach der in Beispiel 14 beschriebenen Arbeitsweise erhält man durch Oxidation von 0,7 g 6-[4,5-Dimethoxy-2-pyridyl)methylthio]-5H-[1,3]-dioxolo[4,5-f]benzimidazol mit 23 ml einer 0,1 m Lösung von m-Chlorperoxybenzoesäure nach Umkristallisation aus Diethylether 0,27 g der Titelverbindung in Form farbloser Kristalle vom F. 199°C (Zers.).

17. 2,2-Difluor-6-[(3,4-dimethoxy-2-pylidyl)methylthio]-5H-[1,3]-dioxolo[4,5-f]benzimidazol

Nach der in Beispiel 13 angegebenen Arbeitsweise erhält man durch Umsetzung von 0,69 g (3 mMol) 2,2-Difluor-5H-[1,3]-dioxolo[4,5-f]benzimidazol-6-thiol mit 0,67 g (3 mMol) 2-Chlormethyl-3,4-dimethoxypyridiniumchlorid in einem Gemisch von 10 ml Ethanol und 10 ml Wasser unter Zusatz von 3,3 ml 2 n Natronlauge nach 10 Stunden Reaktionszeit 1,05 g (92 % d.Th.) der Titelverbindung als feinkristallines, farbloses Pulver vom F. 185 - 187°C.

Analog erhält man
6-[(3,4-Dimethoxy-2-pyridyl)methylthio]-5H-[1,3]-dioxolo[4,5-f]benzimidazol (F. 155 - 157°C)
durch Umsetzung von
5H-[1,3]-dioxolo[4,5-f]benzimidazol-6-thiol
mit
2-Chlormethyl-3,4-dimethoxypyridiniumchlorid.

18. 6-[(4,5-Dimethoxy-3-methyl-3-pyridyl)methylthio]-5H-[1,3]-dioxolo[4,5-f]benzimidazol

0,78 g (4 mMol) 5H-[1,3]-dioxolo[4,5-f]benzimidazol-6-thiol werden mit 0,95 g (4 mMol) 2-Chlormethyl-4,5-dimethoxy-3-methylpyridiniumchlorid in 30 ml Isopropanol 15 Stunden unter Rückfluß zum Sieden erhitzt. Man filtriert vom ausgefallenen Feststoff, rührt mit Isopropanol aus, filtriert erneut und trocknet bis zur Gewichtskonstanz. Man erhält 1,0 g (59 % d.Th.) des Dihydrochlorids der Titelverbindung als farblosen Feststoff vom F. 206°C (Zers.).

19. 2,2-Difluor-6-[(4,5-dimethoxy-2-pyridyl)methylthio]-5H-[1,3]-dioxolo[4,5-f]benzimidazol

Zu einer auf 50°C erwärmten Lösung von 0,69 g 2,2-Difluor-5H-[1,3]-dioxolo[4,5-f]benzimidazol-6-thiol und 0,67 g 2-Chlormethyl-4,5-dimethoxypyridiniumchlorid in 9 ml Ethanol und 4 ml Wasser tropft man innerhalb einer Minute 6,3 ml in Natronlauge zu. Beim Abkühlen der klaren Reaktionsmischung auf 20°C fällt nach kurzer Zeit ein farbloser Niederschlag aus. Man rührt weitere 5 Stunden bei 20°C, saugt über eine Nutsche ab, wäscht mit in Natronlauge und Wasser nach und trocknet bis zur Gewichtskonstanz. Der beige Feststoff wird in 10 ml Methylenchlorid gelöst, von unlöslichen Bestandteilen filtriert, das Filtrat eingeengt und durch Zugabe von Diisopropylether und nach Abkühlung zur Kristallisation gebracht. Man erhält 1,02 g (90 % d.Th.) der Titelverbindung vom F. 189 - 91°C.

Analog erhält man
6,6,7-Trifluor-6,7-dihydro-2-[(4,5-dimethoxy-2-pyridyl)methylthio]-1H-[1,4]-dioxino[2,3-f]benzimidazol,
6-Chlor-6,7,7-trifluor-6,7-dihydro-2-[(4,5-dimethoxy2-pyridyl)methylthio]-1H-[1,4]-dioxino[2,3-f]benzimidazol und
6,7-Dihydro-2-[(4,5-dimethoxy-2-pyridyl)methylthio]-1H-[1,4]-dioxino-[2,3-f]benzimidazol
durch Umsetzung von
6,6,7-Trifluor-6,7-dihydro-1H-[1,4]-dioxino[2,3-f]benzimidazol-2-thiol,
6-Chlor-6,7,7-trifluor-6,7-dihydro-1H-[1,4]-dioxino[2,3-f]benzimidazol-2-thiol, bzw.
6,7-Dihydro-1H-[1,4]-dioxino[2,3-f]benzimidazol-2-thiol
mit
2-Chlormethyl-4,5-dimethoxypyridiniumchlorid.

20. 2,2-Difluor-6-[(4,5-dimethoxy-2-pyridyl)methylsulfinyl)-5H-[1,2]-dioxolo[4,5-f]benzimidazol

0,76 g 2,2-Difluor-6-[(4,5-dimethoxy-2-pyridyl)methylthio]-5H-[1,3]-dioxolo[4,5-f]benzimidazol werden in 10 ml Dioxan und 2 ml 1 n Natronlauge gelöst. Unter Eiskühlung tropft man zuerst eine äquimolare Menge einer titrierten wäßrigen Natriumhypochloritlösung, die mit 1 Mol pro Liter Natronlauge versetzt ist, zu, und setzt nach einer Stunde nochmals 1 Äquivalent und nach 3 Stunden die halbe äquimolare Menge Natriumhypochlorit zur Erreichung einer vollständigen Umsetzung zu. Nach 4 Stunden Reaktionszeit werden 5 ml 5-%-ige Natriumthiosulfatlösung und weitere 25 ml Dioxan zugegeben, die obere Dioxanphase abgetrennt, einmal mit 5 ml Natriumthiosulfatlösung gewaschen und am Rotationsverdampfer eingeengt. Der ölige Rückstand wird in 20 ml Wasser und 10 ml Essigsäureethylester gelöst und mit ca. 100 ml einer Pufferlösung vom pH 6,8 auf pH 7 gestellt. Der ausgefallene Feststoff wird über eine Nutsche abgesaugt, mit Wasser gewaschen, bei 0°C mit Aceton ausgerührt und getrocknet. Man erhält 0,7 g (87 % d.Th.) der Titelverbindung in Form farbloser Kristalle; Zers. bei 211 - 213°C.

Analog erhält man
2,2-Difluor-6-[(3,4-dimethoxy-2-pyridyl)methylsulfinyl]-5H-[1,3]-dioxolo[4,5-f]benzimidazol [F. 177 - 178°C (Zers.)]
6-[(4,5-Dimethoxy-3-methyl-2-pyridyl)methylsulfinyl]-5H-[1,3]-dioxolo[4,5-f]benzimidazol,
6,6,7-Trifluor-6,7-dihydro-2-[(4,5-dimethoxy-2-pyridyl)methylsulfinyl]-1H-[1,4]-dioxino[2,3-f]benzimidazol,
6-[(3,4-Dimethoxy-2-pyridyl)methylsulfinyl]-5H-[1,3]-dioxolo[4,5-f]benzimidazol [F. 170 - 171°C (Zers.)],
6-Chlor-6,7,7-trifluor-6,7-dihydro-2-[(4,5-dimethoxy-2-pyridyl)methylsulfinyl]-1H-[1,4]-dioxino[2,3-f]benzimidazol und
6,7-Dihydro-2-[(4,5-dimethoxy-2-pyridyl)methylsulfinyl]-1H-[1,4]-dioxino[2,3-f]benzimidazol
durch Oxidation der in den Beispielen 17 bis 19 genannten weiteren Sulfide mit Natriumhypochloritlösung.

21. 2-Mercapto-5-(1,1,2,2-tetrafluorethoxy)-1H-benzimidazol

a) 55 g 1-Nitro-4-(1,1,2,2-tetrafluorethoxy)benzol werden in 300 ml Ethanol an 0,5 g 10-%-iger Palladiumkohle in einer Umlaufhydrierungsapparatur unter Atmosphärendruck 1 h bei 20° - 45°C hydriert, der Katalysator abfiltriert und die Lösung bei 40°C im Vakuum eingeengt. Man verdünnt das 4-(1,1,2,2-Tetrafluorethoxy)-anilin mit 100 ml Eisessig und tropft 23 ml Essigsäureanhydrid bei Raumtemperatur zu, versetzt nach 30 Min. mit 2 ml Wasser, engt nach kurzer Zeit die Lösung bei 50°C im Vakuum ein und versetzt mit 500 ml Eiswasser. Man erhält 56 g (97 %) N-[4-(1,1,2,2-Tetrafluorethoxy)phenyl]-acetamid vom Schmp. 121 - 122°C.

b) Man löst 55 g der vorstehenden Verbindung in 380 ml Dichlormethan, tropft 55 ml 100-%-ige Salpetersäure in 10 Min. bei Raumtemperatur zu und rührt noch 6 h. Die organische Lösung wird dann mit wäßriger Natriumcarbonatlösung und Wasser gewaschen, mit Magnesiumsulfat getrocknet und eingeengt. Man erhält 65 g (100 %) N-[2-Nitro-4-(1,1,2,2-Tetrafluorethoxy)phenyl]-acetamid vom Schmp. 80 - 81°C (aus Cyclohexan).

c) Man löst 63 g vorstehender Verbindung in 450 ml Methanol, tropft bei Raumtemperatur 106 ml 6 m Natronlauge zu, kühlt im Eisbad und fällt durch Zutropfen von 900 ml Wasser 53 g (98 %) 2-Nitro-4-(1,1,2,2-tetrafluorethoxy)-anilin (Schmp. 85 - 86°C).

d) 33 g vorstehender Verbindung werden in ca. 600 ml Isopropanol an 1 g 10-%-iger Palladiumkohle in einer Umlaufhydrierungsapparatur drucklos bei Raumtemperatur hydriert. Man saugt den Katalysator ab und fällt mit

4 m Chlorwasserstoff in Ether 34 g (89 %) 4-(1,1,2,2-Tetrafluorethoxy)-1,2-phenylendiamin-dihydrochlorid vom Schmp. 275 - 276° C (Zersetzung).

e) 33 g vorstehender Verbindung werden mit 330 ml Ethanol, 60 ml Wasser, 8,9 g Natriumhydroxid und 23 g Kalium-0-ethyldithiocarbonat (umkristallisiert aus Isopropanol) versetzt und 15 h unter Rückfluß zum Sieden erhitzt. Man versetzt mit 1,2 l Eiswasser, stellt mit Natronlauge auf pH 13 - 14, klärt mit Aktivkohle und fällt mit verdünnter Salzsäure bis pH 3,5. Man erhält 27 g (91 %) der Titelverbindung vom Schmp. 316 - 319° C (aus Isopropanol).

22. 2-Mercapto-5-trifluormethoxy-1H-benzimidazol

Analog Beispiel 21e) erhält man durch Umsetzen von 4-Trifluormethoxy-1,2-phenylendiamin-dihydrochlorid (vgl. C.A. 55, 23408d, 1961) mit Ka-lium-0-ethyldithiocarbonat und Natronlauge in Ethanol in 75 % Ausbeute die Titelverbindung vom Schmp. 305 - 307° C (Zersetzung, aus Toluol).

23. 2-Mercapto-5-(2,2,2-trifluorethoxy)-1H-benzimidazol

a) 50 g 1-(2,2,2-Trifluorethoxy)-4-nitrobenzol (Synthesis 1980, Seite 727) werden analog Beispiel 21a) hydriert und acetyliert. Man erhält 50 g (95 %) N-[4-(2,2,2-Trifluorethoxy)phenyl]-acetamid (Schmp. 140 - 141° C).

b) Man rührt 42 g voranstehender Verbindung mit 9,7 ml 100 % Salpetersäure in 290 ml Eisessig 18 h bei Raumtemperatur und fällt mit Wasser. Man erhält 47 g (94 %) N-[2-Nitro-4-(2,2,2-trifluorethoxy)phenyl]-acetamid (Schmp. 117 - 118° C).

c) Man hydrolysiert 47 g voranstehender Verbindung analog Beispiel 21c und erhält 38,7 g (97 %) 2-Nitro-4-(2,2,2-trifluorethoxy)-anilin (Schmp. 84 - 85° C).

d) Man hydriert 37 g voranstehender Verbindung analog Beispiel 21d) und erhält 41 g (94 %) 4-(2,2,2-Trifluorethoxy)-1,2-phenylendiamin-dihydrochlorid vom Schmp. 230 - 233° C (Zersetzung).

e) Analog Beispiel 21e) erhält man aus 36 g voranstehender Verbindung 30 g (94 %) der Titelverbindung (Schmp. 288 - 290° C).

24. 5-Chlordifluormethoxy-2-mercapto-1H-benzimidazol

a) 10,0 g N-[4-(Chlordifluormethoxy)phenyl]-acetamid (Schmp. 101 - 103° C, aus 4-Chlordifluormethoxyanilin und Essigsäureanhydrid) und 12,3 ml 100 % Salpetersäure werden in 80 ml Dichlormethan 4 h bei 20° C gerührt. Man neutralisiert mit wäßriger Kaliumhydrogencarbonatlösung, engt die organische Schicht ein und erhält 11,4 g (96 %) N-(4-Chlordifluormethoxy-2-nitrophenyl)-acetamid (Schmp. 89 - 91° C).

b) Man tropft bei 5° C zu 10,5 g voranstehender Verbindung in 200 ml Methanol 8,6 ml einer 30-%-igen Lösung von Natriummethylat in Methanol, rührt 2 h ohne Kühlung, versetzt mit Eiswasser, stellt auf pH 8 und erhält 8,7 g (97 %) 4-Chlordifluormethoxy-2-nitroanilin (Schmp. 40 - 42° C).

c) Man hydriert 8,5 g voranstehender Verbindung an 0,8 g 10-%-iger Palladiumkohle drucklos in 200 ml Methanol, versetzt mit konzentrierter Salzsäure, filtriert, engt ein und verrührt mit Diisopropylether. Man erhält 8,5 g (97 %) 4-Chlordifluormethoxy-1,2-phenylendiamin-dihydrochlorid.

d) Aus 8,5 g voranstehender Verbindung werden analog Beispiel 21e) 6,3 g (72 %) der Titelverbindung vom Schmp. 268 - 270° C (Zersetzung) erhalten.

25. 5-Difluormethoxy-2-mercapto-1H-benzimidazol

a) 11,8 g N-(4-Difluormethoxyphenyl)-acetamid [L.M. Jagupol'skii et al., J. General Chemistry (USSR) 39, 190 (1969)] werden in 200 ml Dichlormethan mit 12,1 ml 100-%-iger Salpetersäure 1,5 h bei Raumtemperatur gerührt. Analog Beispiel 21b) erhält man 13,3 g (92 %) N-[(4-Difluormethoxy-2-nitro)phenyl]-acetamid (Schmp. 71 - 73° C).

b) Analog Beispiel 24b) erhält man daraus in 96-%-iger Ausbeute 4-Difluormethoxy-2-nitroanilin (Schmp. 68 - 70° C).

c) Analog Beispiel 24c) erhält man in 94 % Ausbeute 4-Difluormethoxy-1,2-phenylendiamin-dihydrochlorid.

d) Analog Beispiel 21e) erhält man in 78 % Ausbeute die Titelverbindung vom Schmp. 250 - 252° C (aus Isopropanol).

26. 5,6-Bis(difluormethoxy)-2-mercapto-1H-benzimidazol

a) In eine Lösung von 100 g Brenzkatechin, 220 g Natriumhydroxid und 60 g Natriumdithionit in 500 ml Wasser und 400 ml Dioxan leitet man bei 50 - 55° C 275 g Chlordifluormethan analog L.N. Sedova et al., Zh. Org. Khim. 6, 568 (1970) ein. Man erhält nach Destillation bei 61 - 62° C/1,0 - 1,1 kPa eine Mischung von 1,2-Bis(difluormethoxy)Benzol und 2-Difluormethoxyphenol, die durch Chromatographie an Kieselgel mittels Cyclohexan/Essigsäureethylester (4 : 1) getrennt werden.

b) Eine Lösung von 15 g 1,2-Bis(difluormethoxy)benzol und 15 ml 100-%-iger Salpetersäure in 150 ml Dichlormethan wird 7 h bei Raumtemperatur gerührt. Man neutralisiert mit Kaliumhydrogencarbonatlösung, trennt die organische Schicht ab und chromatographiert an Kieselgel mittels Cyclohexan/Essigsäureethylester

(4 : 1). Man erhält 1,2-Bis(difluormethoxy)-4-nitrobenzol. Dieses hydriert und acetyliert man analog Beispiel 21a zu N-[3,4-bis(difluormethoxy)phenyl]acetamid (Schmp. 81 - 83°C). Analog Beispiel 21 erhält man ferner N-[4,5-Bis(difluormethoxy)-2-nitrophenyl]-acetamid (Schmp. 65 - 67°C), N-[4,5-Bis(difluormethoxy)-2-nitro]-anilin (Schmp. 107 - 109°C), 4,5-Bis(difluormethoxy)-1,2-phenylendiamin-dihydrochlorid und die Titelverbindung vom Schmp. 285 - 287°C (Zersetzung: aus Isopropanol).

27. 5-Difluormethoxy-2-mercapto-6-methoxy-1H-benzimidazol

a) In eine Lösung von 55,5 g Guajacol und 130 g Natriumhydroxid in 300 ml Wasser und 300 ml Dioxan werden bei 60°C ca. 58 g Chlordifluormethan eingeleitet. Man filtriert die Mischung bei 10°C, trennt die organische Schicht ab, trocknet mit wasserfreiem Kaliumcarbonat und destilliert. Man erhält 56 g (73 %) 1-Difluormethoxy-2-methoxybenzol vom Siedepunkt 5 - 76°C/0,9 kPa.

b) Zu einer Lösung von 47 g voranstehender Verbindung in 230 ml Dichlormethan wird bei 0 - 5°C eine Lösung von 33,8 ml 100-%-iger Salpetersäure in 90 ml Dichlormethan getropft, nach 30 Min. mit 250 ml Eiswasser versetzt und mit Kaliumhydrogencarbonat neutralisiert. Die getrocknete organische Phase wird im Vakuum eingeengt und der Rückstand aus Cyclohexan umkristallisiert. Man erhält 53 g (90 %) 1-Difluormethoxy-2-methoxy-5-nitrobenzol (Schmp. 48 - 49°C). Dieses wird analog Beispiel 21a hydriert und acetyliert. Man erhält in 90 % Ausbeute N-(3-Difluormethoxy-4-methoxyphenyl)-acetamid (Schmp. 129 - 130°C).

c) 46 g voranstehender Verbindung werden mit 33 ml 100-%-iger Salpetersäure in Dichlormethan analog voranstehender Vorschrift nitriert. Man erhält in 99 % Ausbeute N-(5-Difluormethoxy-4-methoxy-2-nitrophenyl)-acetamid (Schmp. 116 - 117°C).

d) 54 g voranstehender Verbindung werden in 810 ml Methanol 1 h mit 44,8 ml 30-%-iger methanolischer Natriummethylatlösung bei Raumtemperatur gerührt. Man engt im Vakuum ein, versetzt mit Eiswasser und Eisessig bis pH 8 und erhält in 99 % Ausbeute 5-Difluormethoxy-4-methoxy-2-nitroanilin (Schmp. 144 - 145°C).

e) 25 g voranstehender Verbindung werden in 300 ml Methanol an 1,25 g 10-%-iger Palladiumkohle entsprechend Beispiel 21d hydriert. Man erhält 26 g (88 %) 3-Difluormethoxy-4-methoxy-1,2-phenylendiamin-dihydrochlorid vom Schmp. 218 - 220°C (Zersetzung).

f) 25 g voranstehender Verbindung werden mit 19 g Kalium-0-ethyldithiocarbonat entsprechend Beispiel 21e umgesetzt. Man erhält 20 g (89 %) der Titelverbindung vom Schmp. 280 - 282°C (Zersetzung: aus Isopropanol).

28. 5-Difluormethoxy-6-fluor-2-mercapto-1H-benzimidazol

a) Analog Beispiel 27a erhält man aus 2-Fluorphenol und Chlordifluormethan 1-Difluormethoxy-2-fluorbenzol (Sdp. 76°C/10 kPa; $n^{20}_D$ = 1,4340).

b) Zu 30 g der voranstehenden Verbindung in 300 ml Dichlormethan tropft man bei -10°C 38,4 ml 100-%-ige Salpetersäure, rührt 1 h bei -10°C und 2,5 h bei 0°C. Man versetzt mit Eiswasser, stellt neutral und chromatographiert über Kieselgel mit Essigester/Cyclohexan (4 : 1). Man erhält 34 g eines Öles, das ca. 90 % 1-Difluormethoxy-2-fluor-4-nitrobenzol und 10 % 1-Difluormethoxy-2-fluor-5-nitrobenzol (NMR-Spektrum) enthält.

c) 30 g voranstehender Mischung wird analog Beispiel 21a hydriert und acetyliert. Nach Umkristallisieren aus Toluol erhält man 21 g (65 %) N-(4-Difluormethoxy-3-fluorphenyl)-acetamid vom Schmp. 112 - 113°C.

d) Zu 20 g voranstehender Verbindung in 200 ml Dichlormethan werden bei 20°C 22,5 ml 100-%-ige Salpetersäure in 30 Min. zugetropft und 15 h bei Raumtemperatur nachgerührt. Analog Beispiel 27c erhält man in 89 % Ausbeute N-(4-Difluormethoxy-5-fluor-2-nitrophenyl)-acetamid vom Schmp. 72 - 74°C (aus Cyclohexan). Durch mehrstündiges Rühren mit 1 m Salzsäure in Methanol bei 60°C erhält man in 95 % Ausbeute 4-Difluormethoxy-5-fluor-2-nitroanilin vom Schmp. 95 - 97,5°C und analog Beispiel 27e) in 85 % Ausbeute 4-Difluormethoxy-5-fluor-1,2-phenylendiamin-dihydrochlorid. Zersetzung ab 210°C.

e) 15 g voranstehender Verbindung werden mit 11,8 g Kalium-0-ethyldithiocarbonat entsprechend Beispiel 21e umgesetzt. Man erhält 11,1 g (84 %) der Titelverbindung vom Schmp. 275 - 276°C (Zersetzung, aus Isopropanol).

29. 2,2-Difluor-5H-[1,3]-dioxolo[4,5-f]benzimidazol-6-thiol

a) Man hydriert 30 g 4-Amino-2,2-difluor-5-nitro-1,3-benzodioxol in 300 ml Methanol an 0,5 g 10-%-iger Palladiumkohle in einer Umlaufhydrierungsappatur bei Atmosphärendruck und Raumtemperatur, versetzt mit 2,5 Äquivalenten methanolischer Chlorwasserstofflösung, filtriert, engt die Lösung im Vakuum ein, versetzt mit Isopropanol und Ether und erhält 35 g (97 %) 2,2-Difluor-1,3-benzodioxol-4,5-diamin-dihydrochlorid vom Schmp. 232 - 233°C (Zersetzung).

b) Man versetzt 30 g voranstehender Verbindung in 300 ml Ethanol mit 24 g Kalium-0-ethyldithiocarbonat (umkristallisiert aus Isopropanol) und 9,2 g Natriumhydroxid in 55 ml Wasser und erhitzt 15 h unter Rückfluß zum Sieden. Man gießt auf 1,5 l Wasser, stellt mit Natronlauge auf pH 14, klärt mit Aktivkohle, fällt mit konzentrierter Salzsäure in der Wärme und saugt den Niederschlag in der Kälte ab. Man erhält 24 g (91 %) der Titelverbindung vom Schmp. 365 - 370°C (Zersetzung).

30. 6,6,7-Trifluor-6,7-dihydro-1H-[1,4]-dioxino[2,3-f]benzimidazol-2-thiol

a) Zu 50 g 2,2,3-Trifluor-2,3-dihydro-1,4-benzodioxin wird bei 5°C in 1 h eine Mischung von 39,5 ml 69-%-iger Salpetersäure und 46 ml 97-%-iger Schwefelsäure getropft. Man rührt 1 h bei 10°C, 1 h bei 20°C und 5 Min. bei 40°C und gießt auf 200 g Eis, extrahiert mit Dichlormethan, wäscht mit Wasser, trocknet mit Magnesiumsulfat und destilliert im Vakuum. Man erhält 58 g (94 %) einer Mischung von 2,2,3-Trifluor-2,3-dihydro-6-nitro-(und 7-nitro)-1,4-benzodioxin vom Sdp. 68,5°C (0,15 mbar) und $n^{20}_D$ 1,5080. Ein Gaschromatogramm mit einer 10 m Fused Silica Säule (Fa. Chrompack) zeigt zwei Peaks im Verhältnis 2 : 3.

b) Man hydriert 35 g des Isomerengemisches in 400 ml Ethanol an 3 g 10-%-iger Palladiumkohle bei Atmosphärendruck und 20 - 30°C in einer Umlaufhydrierungsapparatur, filtriert und engt im Vakuum ein. Man erhält 30,5 g (100 %) einer flüssigen Mischung von 6-Amino-(und 7-Amino)-2,2,3-trifluor-2,3-dihydro-1,4-benzodioxin.

c) Zu 28 g der voranstehenden Isomerenmischung tropft man bei 20 - 30°C eine Mischung aus 15,3 g Essigsäureanhydrid und 15 ml Eisessig, rührt 30 Min. bei 30°C, setzt 1 ml Wasser zu, rührt 30 Min. bei 30°C und destilliert das Lösungsmittel im Vakuum ab. Durch Umkristallisation aus Toluol erhält man 19 g einer Fraktion des Gemisches der isomeren Acetaminoderivate vom Schmp. 128 - 133°C.

d) Zu 17 g des Isomerengemisches der Acetaminoderivate, suspendiert in 200 ml Dichlormethan, tropft man bei -6° bis -8°C 14 ml 100-%-ige Salpetersäure, gelöst in 60 ml Dichlormethan, rührt 2 h bei 0°C und dann über Nacht bei Raumtemperatur. Man gießt auf 110 g Eis, trennt die organische Phase ab, wäscht mit Wasser und engt im Vakuum ein. Der Rückstand (19,8 g) wird aus 20 ml Ethanol umkristallisiert. Man erhält 15,5 g einer Mischung von 6-Acetamino-2,2,3-trifluor-2,3-dihydro-7-nitro-1,4-benzodioxin und 7-Acetamino-2,2,3-trifluor-2,3-dihydro-6-nitro-1,4-benzodioxin.

e) Man suspendiert 14,5 g des voranstehenden Produktgemisches in 80 ml Methanol und tropft unter Erwärmung auf 30°C 30 ml 5 m Natronlauge zu. Man rührt noch 0,5 h bei Raumtemperatur, gießt auf 200 g Eis und erhält 11,7 g einer Mischung von 6-Amino-2,2,3-trifluor-2,3-dihydro-7-nitro-1,4-benzodioxin und 7-Amino-2,2,3-trifluor-2,3-dihydro-6-nitro-1,4-benzodioxin. Eine Probe wird an einer Kieselgelsäule mit Cyclohexan /Essigsäureethylester (4 : 1) in zwei reine Isomeren mit den Schmelzpunkten 110,5 - 111,5°C und 120 - 121°C getrennt, deren NMR-Spektren an einem 60 MHz-Gerät in Deuterochloroform praktisch identisch sind.

f) 10,9 g des voranstehenden Isomerengemisches werden in 300 ml Methanol bei Raumtemperatur und Atmosphärendruck an 1 g 10-%-iger Palladiumkohle in 2,5 h hydriert. Man setzt 30 ml 4 m Chlorwasserstoff in Methanol zu, filtriert, engt im Vakuum ein und verrührt mit 100 ml Ether. Man erhält 12,6 g (98 %) 2,2,3-Trifluor-2,3-dihydro-1,4-benzodioxin-6,7-diamindihydrochlorid (Schmp. >250°C).

g) 12 g voranstehender Verbindung und 8,5 g Kalium-0-ethyldithiocarbonat (umkristallisiert aus Isopropanol) werden in 120 ml Ethanol mit 20,5 ml 4 m wäßriger Kaliumhydroxidlösung versetzt und 17 h unter Rückfluß zum Sieden erhitzt. Man gießt auf 300 g Eis, stellt mit Kaliumhydroxidlösung auf pH 12 - 13, klärt mit Aktivkohle und fällt mit konzentrierter Salzsäure. Nach erneuter Fällung mit Säure aus alkalischer wäßrig-alkoholischer Lösung erhält man 10 g (93 %) der Titelverbindung vom Schmp. 309 - 310°C (Zersetzung).

31. 6-Chlor-6,7,7-trifluor-6 7-dihydro-1H-[1,4]-dioxino[2,3-f]benzimidazol-2-thiol

a) Zu 18 g 2-Chlor-2,3,3-trifluor-2,3-dihydro-1,4-benzodioxin tropft man bei 5°C eine Mischung von 18,3 ml 65-%-iger Salpetersäure und 15,4 ml 97-%-ige Schwefelsäure, rührt 2 h bei 5 - 10°C und gießt auf Eis. Man extrahiert mit Methylenchlorid und erhält 21,3 g einer Mischung von 2-Chlor-2,3,3-trifluor-2,3-dihydro-6-nitro-(und 7-nitro)-1,4-benzodioxin als Öl.

b) Analog Beispiel 30b) erhält man daraus in 95 % Ausbeute eine ölige Mischung von 2-Chlor-2,3,3-trifluor-2,3-dihydro-1,4-benzodioxin-6-(und 7-)amin, welche entsprechend Beispiel 30c) zu einer Mischung der entsprechenden Acetaminoderivate quantitativ umgesetzt wird.

c) 19 g der voranstehenden Mischung wird in 190 ml Dichlormethan mit 16 ml 100-%-iger Salpetersäure gerührt und das Reaktionsprodukt durch Chromatographie an Kieselgel mittels Cyclohexan/Essigsäureethylester (4 : 1) gereinigt. Man erhält 15 g einer Mischung von 6-Acetamino-2-chlor-2,3,3-trifluor-6,7-dihydro-7-nitro-1,4-benzodioxin und 7-Acetamino-2-chlor-2,3,3-trifluor-6,7-dihydro-6-nitro-1,4-benzodioxin als hellgelbes Öl.

d) Zu 14,5 g der voranstehenden Mischung in 100 ml Methanol tropft man bei 5°C 10,2 ml einer 30-%-igen Lösung von Natriummethylat in Methanol, rührt 1,5 h ohne Kühlung, gießt auf Eis, neutralisiert mit verdünnter Salzsäure, extrahiert mit Dichlormethan und engt im Vakuum ein. Man erhält 12,7 g einer Mischung von 6-Amino-2-chlor-2,3,3-trifluor-2,3-dihydro-7-nitro-1,4-benzodioxin und 7-Amino-2-chlor-2,3,3-trifluor-2,3-dihydro-6-nitro-1,4-benzodioxin als orangefarbenes Öl.

e) 12,4 g der voranstehenden Mischung werden analog Beispiel 30f) hydriert. Man erhält 12,6 g (99 %) 2-Chlor-2,3,3-trifluor-2,3-dihydro-1,4-benzodioxin-6,7-diamin-dihydrochlorid.

f) 12,4 g der voranstehenden Verbindung werden analog Beispiel 30g) mit 9,1 g Kalium-0-ethyldithiocarbonat und Kaliumhydroxidlösung in Ethanol umgesetzt. Man erhält 9,6 g (74 %) der Titelverbindung vom Schmp. 288 - 290°C (Zersetzung).

32. 2-Chlormethyl-4,5-dimethoxy-pyridinium-chlorid

a) 2-Chlormethyl-4,5-dimethoxy-pyridinium-chlorid

Zu einer auf 0°C gekühlten Lösung von 5 g 2-Hydroxymethyl-4,5-dimethoxypyridin in 40 ml Methylenchlorid tropft man innerhalb einer Stunde 3 ml Thionylchlorid, gelöst in 10 ml Methylenchlorid zu, rührt anschließend 4 Stunden bei 20°C, wobei sich die Reaktionsmischung rot färbt, setzt 5 ml Toluol zu und engt am Rotationsverdampfer vollständig ein (30°C/5 mbar). Der ölige Rückstand wird in 50 ml warmem Isopropanol gelöst, mit wenig Tonsil® geklärt, filtriert und erneut eingeengt. Man nimmt in 10 ml Toluol auf und bringt die Lösung mit Petrolether zur Kristallisation. Nach Abkühlung im Eisbad wird abgesaugt, mit Petrolether gewaschen und getrocknet. Man erhält 4,6 g (70 % d.Th.) der Titelverbindung 2-Chlormethyl-4,5-dimethoxy-pyridinium-chlorid als farblosen Feststoff; Zers. bei 160 - 61°C.

b) 2-Hydroxymethyl-4,5-dimethoxy-pyridin

19 g 4,5-Dimethoxy-2-methylpyridin-1-oxid werden innerhalb von 30 Minuten in der Weise zu 60 ml auf 80°C erwärmten Essigsäureanhydrid zudosiert, daß die Temperatur nicht über 100°C steigt. Nach weiteren 45 Minuten bei 85°C wird überschüssiges Essigsäureanhydrid im Vakuum abdestilliert und der ölige dunkle Rückstand, der im wesentlichen aus dem Zwischenprodukt 2-Acetoxymethyl-4,5-dimethoxypyridin besteht, mit 80 ml 2 n Natronlauge 1 Stunde lang bei 80°C gerührt. Nach Verdünnen mit 80 ml Wasser und Abkühlung wird achtmal mit je 100 ml Methylenchlorid extrahiert, die vereinigten organischen Phasen zweimal mit 1 n Natronlauge gewaschen, getrocknet, eingeengt und der kristalline, bräunliche Rückstand aus Toluol umkristallisiert. Man erhält 14 g (74 % d.Th.) 2-Hydroxymethyl-4,5-dimethoxy-pyridin vom F. 122 - 24°C.

c) 4,5-Dimethoxy-2-methylpyridin-1-oxid

Zu einer Suspension von 16,9 g 5-Methoxy-2-methyl-4-nitropyridin-1-oxid in 170 ml trockenem Methanol werden 20 ml einer 30-%-igen Natriummethylatlösung zugetropft, 15 Stunden bei 20°C und anschließend 4 Stunden bei 50°C gerührt. Man stellt durch vorsichtige Zugabe von konzentrierter Schwefelsäure unter Eiskühlung auf pH 7, engt ein, rührt den Rückstand mit 200 ml Methylenchlorid aus, filtriert von unlöslichen Bestandteilen, versetzt mit 10 ml Toluol und engt erneut zur Trockne ein. Man erhält 15,2 g (98 % d.Th.) 4,5-Dimethoxy-2-methylpyridin-1-oxid als farbloses Kristallisat vom F. 118 - 121°C.

d) 5-Methoxy-2-methyl-4-nitropyridin-1-oxid

Zu 35 ml auf 60°C erwärmte 65-%-ige Salpetersäure werden 21,2 g 5-Methoxy-2-methylpyridin-1-oxid in der Weise zudosiert, daß die Temperatur der Reaktionsmischung 80°C nicht übersteigt. Man rührt 1 Stunde bei 80°C, setzt zur vollständigen Umsetzung noch 13 ml 100-%-ige Salpetersäure zu und rührt weitere 2 Stunden bei 60 - 70°C. Zur Aufarbeitung gießt man auf 300 g Eis. Der ausgefallene gelbe Niederschlag wird über ein Nutsche filtriert, mit Wasser gewaschen und getrocknet. Der trockene Feststoff wird mit 200 ml Methylenchlorid ausgekocht, filtriert und getrocknet. Durch Konzentrierung des Filtrats wird weiteres DC-reines Produkt isoliert. Man erhält 22,3 g (87 % d.Th.) 5-Methoxy-2-methyl-4-nitropyridin-1-oxid vom F. 201 - 202°C; gelbe Kristalle.

e) 5-Methoxy-2-methylpyridin-1-oxid

Zu einer Lösung von 60,9 g 5-Methoxy-2-methylpyridin in 300 ml Eisessig werden bei 60°C 120 g 30-%-ige Wasserstoffperoxidlösung innerhalb von 1 Stunde zugetropft und 3 Stunden nachgerührt. Nach Zerstörung von überschüssigen Perverbindungen durch Zugabe von aktivem Mangandioxid wird filtriert, eingeengt, der Rückstand in 500 ml Essigsäureethylester heiß geklärt, erneut eingeengt und bei 0,3 mbar destilliert. Man erhält 54 g (77 % d.Th.) 5-Methoxy-2-methylpyridin-1-oxid als rasch erstarrendes Öl (Sdp. 130°C); F. 80 - 84°C.

f) 5-Methoxy-2-methylpyridin

Zu einer Lösung von 84 g Kaliumhydroxid in 400 ml Methanol und 500 ml Dimethylsulfoxid werden innerhalb einer Stunde 150 ml 3-Hydroxy-6-methylpyridin zudosiert. Nach Entfernung des Methanols am Rotationsverdampfer tropft man unter Eiskühlung 213 g Methyliodid, gelöst in 100 ml Dimethylsulfoxid zu, rührt 15 Stunden bei 20°C und unterwirft das Reaktionsgemisch einer Wasserdampfdestillation. Das Destillat wird am Extraktor kontinuierlich mit Methylenchlorid extrahiert und der Extrakt eingeengt. Man erhält 85 g (56 % d.Th.) 5-Methoxy-2-methylpyridin als farbloses Öl.

33. 2-Chlormethyl-4,5-dimethoxy-3-methylpyridinium-chlorid

a) 2-Chlormethyl-4,5-dimethoxy-3-methylpyridinium-chlorid

Nach der in Beispiel 32a) beschriebenen Arbeitsweise erhält man durch Umsetzung von 2,7 g 2-Hydroxymethyl-4,5-dimethoxy-3-methylpyridin mit 4 g Thionylchlorid in 25 ml Methylenchlorid nach 1 Stunde Reaktionszeit und nach einer vereinfachten Aufarbeitungsmethode, nämlich durch Zusatz von 10 ml Toluol, Abdestillieren des Methylenchlorids und überschüssigen Thionylchlorids, Absaugung des ausgefallenen Kristallisats und Trocknung 3,45 g (99 % d.Th) der Titelverbindung als farblose Kristalle; Zers. bei 125 - 26°C.

b) 2-Hydroxymethyl-4,5-dimethoxy-3-methylpyridin

4,5 g 4,5-Dimethoxy-2,3-dimethylpyridin-1-oxid werden in 20 ml Essigsäureanhydrid 30 Minuten auf 110°C erwärmt und anschließend am Rotationsverdampfer eingeengt. Der ölige Rückstand, der aus dem Zwischenprodukt 2-Acetoxymethyl-4,5-dimethoxy-3-methylpyridin besteht, wird in 30 ml 3 n Natronlauge 2 Stunden bei 80°C gerührt, nach Abkühlung fünfmal mit je 30 ml Methylenchlorid extrahiert, die vereinigten organischen Phasen zweimal mit 2 n Natronlauge gewaschen, getrocknet, eingeengt, der Rückstand mit Petrolether verrührt, abgesaugt und getrocknet. Man erhält 4,0 g (89 % d.Th.) 2-Hydroxymethyl-4,5-dimethoxy-3-methylpyridin vom F. 91 - 92°C.

c) 4,5-Dimethoxy-2,3-dimethylpyridin-1-oxid

6,3 g 4,5-Dimethoxy-2,3-dimethylpyridin werden in 120 ml Methylenchlorid gelöst, sukzessive 20 g m-Chlorperoxybenzoesäure zugegeben, erst 2 Stunden bei 20°C und anschließend 4 Stunden bei 40°C gerührt. Nach Zusatz von 20 ml 5 n Natronlauge wird dreimal mit einem Gemisch aus einer 5-%-igen Natriumthiosulfat- und 5-%-igen Natriumcarbonatlösung gewaschen, die Wasserphasen zweimal mit Methylenchlorid rückextrahiert, die vereinigten organischen Phasen über Magnesiumsulfat getrocknet und eingeengt. Man erhält 4,6 g (66 % d.Th.) 4,5-Dimethoxy-2,3-dimethylpyridin-1-oxid. Der Rf-Wert in Methylenchlorid/Methanol 19 : 1 beträgt 0,25.

d) 4,5-Dimethoxy-2,3-dimethylpyridin

Nach der in Beispiel 32f) beschriebenen Arbeitsweise erhält man durch Umsetzung von 9,18 g 5-Hydroxy-4-methoxy-2,3-dimethylpyridin in 50 ml Dimethylsulfoxid zuerst mit 3,6 g Natriumhydroxid, anschließend mit 8,95 g Methyliodid 7,4 g (74 % d.Th.) 4,5-Dimethoxy-2,3-dimethylpyridin als farbloses, allmählich kristallisierendes Öl, F. 36 - 38°C.

e) 5-Hydroxy-4-methoxy-2,3-dimethylpyridin

1000 g 4-Methoxy-2,3-dimethylpyridin-1-oxid werden bei 100°C unter Rühren innerhalb von 7 Stunden zu 3 l Essigsäureanhydrid zudosiert und weitere 3 Stunden bei 100°C nachgerührt. Man läßt abkühlen, engt bei 70°C/10 mbar vollständig ein und destilliert anschließend bei $10^{-2}$ mbar. Die Fraktion mit einem Siedeintervall von 95 - 145°C (Gemisch aus dem Zwischenprodukt 5-Acetoxy-4-methoxy-2,3-dimethylpyridin und 2-Acetoxymethyl-4-methoxy-3-methylpyridin) wird abgenommen (952 g) und zu 3,5 l auf 50°C erwärmte 2 n Natronlauge innerhalb von 30 Minuten zugegeben.

Man rührt bei 50°C bis zur Bildung einer klaren Lösung (ca. 3 Stunden), läßt abkühlen und extrahiert dreimal mit je 1 l Methylenchlorid. Die vereinigten organischen Phasen werden zweimal mit je 0,5 l 1 n Natronlauge rückextrahiert und anschließend die vereinigten Wasserphasen mit halbkonzentrierter Salzsäure unter Rühren auf pH 7,5 gestellt. Man filtriert vom ausgefallenen Feststoff, wäscht nach und trocknet bis zur Gewichtskonstanz. Man erhält 5-Hydroxy-4-methoxy-2,3-dimethylpyridin vom F. 274 - 76°C.

34. <u>2-Chlormethyl-3 4-dimethoxy-pyridinium-chlorid</u>

a) 2-Chlormethyl-3,4-dimethoxy-pyridiniumchlorid

Nach der in Beispiel 32a beschriebenen Arbeitsweise erhält man durch Umsetzung von 3,38 g 2-Hydroxymethyl-3,4-dimethoxypyridin mit 2 ml Thionylchlorid in 30 ml Methylenchlorid nach 2,5 Stunden Reaktionszeit und nach der in Beispiel 33a beschriebenen Art der Aufarbeitung 4,2 g (93 % d.Th.) der Titelverbindung als farblosen Feststoff vom F. 151 - 152°C (Zers.)

b) 2-Hydroxymethyl-3,4-dimethoxypyridin

4,8 g 2-Acetoxymethyl-3,4-dimethoxypyridin werden nach Zusatz von 15 ml 2 n Natronlauge bei 80°C kräftig gerührt, wobei sich aus dem anfänglichen Zweiphasengemisch eine homogene Lösung bildet. Nach 2 h läßt man abkühlen, extrahiert fünfmal mit je 30 ml Methylenchlorid, wäscht die vereinigten organischen Phasen zweimal mit je 5 ml 0,3 n Natronlauge, trocknet über Kaliumcarbonat, filtriert, engt ein und verrührt den Destillationsrückstand mit Petrolether. Man erhält 3,6 g (96 % d.Th.) 2-Hydroxymethyl-3,4-dimethoxypyridin als farblosen Feststoff vom F. 87 - 89°C.

c) 2-Acetoxymethyl-3,4-dimethoxypyridin

Zu 25 ml Essigsäureanhydrid werden bei 85°C innerhalb von einer Stunde 4,8 g (28 mMol) 3,4-Dimethoxy-2-methylpyridin-1-oxid zudosiert, eine Stunde bei derselben Temperatur gerührt, im Vakuum vollständig eingeengt und der braune, ölige Rückstand in einer Kugelrohrdestille bei 1 Pa destilliert. Man erhält 5,3 g (90 % d.Th.) 2-Acetoxymethyl-3,4-dimethoxypyridin; Sdp. 125 - 130°C.

d) 3,4-Dimethoxy-2-methylpyridin-1-oxid

4,5 g (25 mMol) 3-Methoxy-2-methyl-4-nitropyridin-1-oxid werden in 75 ml trockenem Methanol nach Zusatz von 4,7 ml 30-%-iger Natriummethylatlösung 16 Stunden bei 40°C gerührt. Anschließend kühlt man ab, stellt mit konz. Schwefelsäure auf pH 7, filtriert, engt im Vakuum vollständig ein, nimmt den öligen, rötlichen Rückstand in 50 ml Toluol auf, filtriert erneut von unlöslichen Bestandteilen und engt das Filtrat zur Trockene ein. Der gelbe, ölige Rückstand kristallisiert im Eisbad und wird abschließend mit 30 ml Petrolether (50/70) bei 40°C ausgerührt. Nach Filtration und Trocknung im Exsiccator erhält man 5,2 g (88 % d.Th.) 3,4-Dimethoxy-2-methylpyridin-1-oxid in Form blaßgelber Kristalle vom F. 111 - 113°C.

e) 3-Methoxy-2-methyl-4-nitropyridin-1-oxid

Zu 5,4 g 3-Methoxy-2-methylpyridin-1-oxid in 12 ml Eisessig werden bei 80°C innerhalb von 6 h in vier Portionen von je 2 ml 8 ml konz. Salpetersäure zugegeben, über Nacht bei derselben Temperatur gerührt, nochmals in drei Portionen innerhalb von 6 Stunden 8 ml Salpetersäure zugegeben und weitere 15 Stunden gerührt. Nach Abkühlung gießt man auf Eis (40 g), stellt mit 10 n Natronlauge auf pH 6, filtriert vom ausgefallenen Nebenprodukt (3-Methoxy-2-methyl-4-nitropyridin) und extrahiert viermal mit 50 ml Methylenchlorid. Nach Trocknung werden die vereinigten organischen Phasen vollständig eingeengt und der Rückstand aus wenig Methylenchlorid/Petrolether umkristallisiert. Man erhält 4,2 g (57 % d.Th.) der Titelverbindung in Form gelber Kristalle vom F. 103 - 104°C.

f) 3-Methoxy-2-methylpyridin-1-oxid

15,3 g (0,124 Mol) 3-Methoxy-2-methylpyridin werden in 100 ml Eisessig gelöst und bei 80°C in 4 Portionen 40 ml 30-%-iges Wasserstoffperoxid innerhalb von 6 Stunden zugegeben. Man rührt weitere drei Stunden und engt anschließend im Vakuum (1,5 kPa) ein, setzt zweimal je 50 ml Essigsäure zu und engt jeweils vollständig ein. Nach negativem Nachweis auf Perverbindungen wird im Kugelrohrofen destilliert. Die bei 120°C (1,5 Pa)

destillierende Fraktion wird in wenig Diethylether ausgerührt, der Feststoff filtriert und getrocknet. Man erhält 12 g (60 % d.Th.) 3-Methoxy-2-methylpyridin-1-oxid in Form farbloser Kristalle vom F. 72 - 78° C.

g) 3-Methoxy-2-methylpyridin

Nach der in Beispiel 32f beschriebenen Arbeitsweise erhält man durch Umsetzung von 13,7 g (125 mMol) 3-Hydroxy-2-methylpyridin mit 9,2 ml Methyliodid unter Zusatz von 46 ml 3 m methanolischer Kaliumhydroxidlösung nach einer Reaktionszeit von 3 Stunden 15,5 g (90 % d.Th.) 3-Methoxy-2-methylpyridin als farbloses Öl.

**Gewerbliche Anwendbarkeit**

Die Dialkoxypyridine der allgemeinen Formel I und ihre Salze besitzen wertvolle pharmakologische Eigenschaften, die sie gewerblich verwertbar machen. Sie hemmen deutlich die Magensäuresekretion von Warmblütern und weisen darüberhinaus eine ausgezeichnete Magen- und Darmschutzwirkung bei Warmblütern auf. Diese Magen- und Darmschutzwirkung wird bereits bei der Verabreichung von Dosen beobachtet, die unterhalb der säuresekretionshemmenden Dosen liegen. Darüberhinaus zeichen sich die erfindungsgemäßen Verbindungen durch das Fehlen wesentlicher Nebenwirkungen und eine große therapeutische Breite aus.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z. B. Ulcus ventriculi, Ulcus duodeni, Gastritis, hyperazider oder medikamentös bedingter Reizmagen) verstanden, die beispielsweise durch Mikroorganismen, Bakterientoxine, Medikamente (z. B. bestimmte Antiphlogistika und Antirheumatika), Chemikalien (z. B. Ethanol), Magensäure oder Streßsituationen verursacht werden können.

Ein weiterer Vorteil der erfindungsgemäßen Verbindungen ist ihre vergleichsweise große chemische Stabilität.

In ihren ausgezeichneten Eigenschaften erweisen sich die erfindungsgemäßen Verbindungen überraschenderweise den aus dem Stand der Technik bekannten Verbindungen deutlich überlegen. Aufgrund dieser Eigenschaften sind die Dialkoxypyridine und ihre pharmakologisch verträglichen Salze für den Einsatz in der Human- und Veterinärmedizin hervorragend geeignet, wobei sie insbesondere zur Behandlung und/oder Prophylaxe von Krankheiten des Magens und Darms und solcher Krankheiten, die auf einer überhöhten Magensäuresekretion beruhen, verwendet werden.

Ein weiterer Gegenstand der Erfindung sind daher die erfindungsgemäßen Verbindungen zur Anwendung bei der Behandlung und/oder Prophylaxe der vorstehend genannten Krankheiten.

Ebenso umfaßt die Erfindung die Verwendung der erfindungsgemäßen Verbindungen zur Herstellung von Arzneimitteln, die zur Behandlung und/oder Prophylaxe der vorstehend genannten Krankheiten eingesetzt werden.

Ein weiterer Gegenstand der Erfindung sind Arzneimittel, die ein oder mehrere Dialkoxypyridine der allgemeinen Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoffe) entweder als solche, oder vorzugsweise in Kombination mit geeigneten pharmazeutischen Hilfs- oder Trägerstoffen in Form von Tabletten, Dragees, Kapseln, Suppositorien, Pflastern (z. B. als TTS), Emulsionen, Suspensionen oder Lösungen eingesetzt, wobei der Wirkstoffgehalt vorteilhafterweise zwischen 0,1 und 95 % beträgt.

Welche Hilfs- bzw. Trägerstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z. B. Cyclodextrine) verwendet werden.

Die Wirkstoffe können oral, parenteral oder percutan appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 20, vorzugsweise 0,05 bis 5, insbesondere 0,1 bis 1,5 mg/kg Körpergewicht, gegebenenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder Salze zur Behandlung der oben genannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat; Tranquilizer, wie Benzodiazpine, beispielsweise Diazepam; Spasmolytika, wie z. B. Bietamiverin, Camylofin; Anticholinergica, wie z. B. Oxyphencyclimin, Phencarbamid; Lokalanaesthetika, wie z. B. Tetracain, Procain; gegebenenfalls auch Fermente, Vitamine oder Aminosäuren enthalten.

Hervorzuheben ist in diesem Zusammenhang insbesondere die Kombination der erfindungsgemäßen

Verbindungen mit anderen, die Säuresekretion hemmenden Pharmaka, wie beispielsweise $H_2$-Blockern (z. B. Cimetidin, Ranitidin), ferner mit sogenannten peripheren Anticholinergika (z. B. Pirenzepin, Telenzepin, Zolenzepin) sowie mit Gastrin-Antagonisten, mit dem Ziel, die Hauptwirkung in additivem oder überadditivem Sinn zu verstärken und/oder die Nebenwirkungen zu eliminieren oder zu verringern.

**Pharmakologie**

Die ausgezeichnete Magenschutzwirkung und die magensekretionshemmende Wirkung der erfindungsgemäßen Verbindungen läßt sich tierexperimentell am Modell Shay-Ratte nachweisen. Die untersuchten erfindungsgemäßen Verbindungen sind wie folgt mit Nummern versehen worden:

| Lfd. Nr. | Name der Verbindung |
|---|---|
| 1 | 2-[(4,5-Dimethoxy-2-pyridyl)methylsulfinyl]-5-trifluormethoxy-1H-benzimidazol |
| 2 | 2-[(4,5-Dimethoxy-3-methyl-2-pyridyl)methylsulfinyl]-5-trifluormethoxy-1H-benzimidazol |
| 3 | 2-[(4,5-Dimethoxy-2-pyridyl)methylsulfinyl]-5-(1,1,2,2-tetrafluorethoxy)-1H-benzimidazol |
| 4 | 2,2-Difluor-6-[(4,5-dimethoxy-2-pyridyl)methylthio]-5H-[1,3]-dioxolo[4,5-f]benzimidazol |
| 5 | 2,2-Difluor-6-[(4,5-dimethoxy-2-pyridyl)methylsulfinyl]-5H-[1,3]-dioxolo[4,5-f]benzimidazol |

Der Einfluß der untersuchten Verbindungen auf die durch Pylorusligatur (4 h; sog. Shay-Ratte) und orale Gabe von 100 mg/kg Acetylsalicylsäure ausgelöste Magenläsionsbildung sowie die Magensekretion (HCl) während 4 h bei der Ratte, ist in der folgenden Tabelle dargestellt.

**Magenschutzwirkung und Magensekretionshemmung**

| Lfd. Nr. | n [Anzahl d. Tiere] | Magenschutzwirkung (Ratte) Hemmung d. Läsionsindexes, ED50*) [mg/kg, p.o.] | Hemmung der HCl-Sekretion d. Magens (Ratte; Summe 4 h) % Hemmung der HCl-Sekretion **) | ED25*) [mg/kg, p.o.] | ED50*) [mg/kg, p.o.] |
|---|---|---|---|---|---|
| 1 | 40 | 0,6 | 15 | 1,0 | ~ 3 |
| 2 | 48 | 0,8 | 25 | 0,7 | 1,7 |
| 3 | 56 | 0,6 | 18 | ~ 1 | 3,4 |
| 4 | 40 | 3,5 | 28 | 3,0 | 6,5 |
| 5 | 72 | ~ 1 | 25 | 1,0 | 3,0 |

*) ED25 bzw. ED50 = Dosis, die den Läsionsindex bzw. die HCl-Sekretion (4 h) des Rattenmagens bei der behandelten Gruppe gegenüber der Kontrollgruppe um 25 bzw. 50 % mindert.
**) nach Gabe der antiulcerösen ED50

Die Prüfung der antiulcerogenen Wirkung erfolgte nach der Methode der sogenannten Shay-Ratte:

Die Ulcusprovokation erfolgt bei 24 Stunden nüchtern gehaltenen Ratten (weiblich, 180 - 200 g, 4 Tiere je Käfig auf hohem Gitterrost) durch Pylorusligatur (unter Diethylethernarkose) und orale Applikation von 100 mg/10 ml/kg Acetylsalicylsäure. Die zu prüfenden Substanzen werden oral (10 ml/kg) eine Stunde vor der Pylorusligatur verabreicht. Der Wundverschluß wird mittels Michelklammern vorgenommen. 4 Stunden danach erfolgt die Tötung der Tiere im Etherrausch durch Atlas-Dislokation und die Resektion des Magens. Der Magen wird längs eröffnet und auf einer Korkplatte fixiert, nachdem zuvor die Menge des sezernierten Magensaftes (Volumen) und später sein HCl-Gehalt (Titration mit Natronlauge) bestimmt wurde; mit einem Stereomikroskop werden bei 10-facher Vergrößerung Anzahl und Größe (= Durchmesser) vorhandener Ulcera ermittelt. Das Produkt aus Schweregrad (gemäß nachfolgender Punkteskala) und Anzahl der Ulcera dient als individueller Läsionsindex.

Punkteskala:

| | | |
|---|---|---|
| keine Ulcera | | 0 |
| Ulcusdurchmesser | 0,1 - 1,4 mm | 1 |
| | 1,5 - 2,4 mm | 2 |
| | 2,5 - 3,4 mm | 3 |
| | 3,5 - 4,4 mm | 4 |
| | 4,5 - 5,4 mm | 5 |
| | >5,5 mm | 6 |

Als Maß für den antiulcerogenen Effekt dient die Minderung des mittleren Läsionsindex jeder behandelten Gruppe gegenüber dem der Kontrollgruppe (= 100 %). Die ED25 bzw. ED50 bezeichnen diejenigen Dosen, die den mittleren Läsionsindex bzw. die HCl-Sekretion gegenüber der Kontrolle um 25 % bzw. 50 % mindern.

**Toxizität**

Die LD50 aller geprüften Verbindungen liegt oberhalb von 1000 mg/kg [p.o.] bei der Maus.

**Patentansprüche** für die Vertragsstaaten: BE, CH, DE, FR, GB, IT, LI, LU, NL, SE

1. Dialkoxypyridine der allgemeinen Formel I

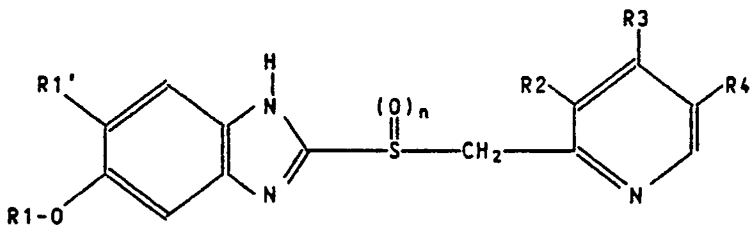

(I),

worin

R1 einen ganz oder überwiegend durch Fluor substituierten 1-3C-Alkylrest oder einen Chlordifluormethylrest und

R1' ein Wasserstoff- oder Halogenatom, Trifluormethyl, einen 1-3C-Alkylrest oder einen gegebenenfalls ganz oder überwiegend durch Fluor substituierten 1-3C-Alkoxyrest oder

R1 und R1' gemeinsam und unter Einschluß des Sauerstoffatoms, an das R1 gebunden ist, einen gegebenenfalls ganz oder teilweise durch Fluor substituierten 1-2C-Alkylendioxyrest oder einen Chlortrifluorethylendioxyrest darstellen,

R3 einen 1-3C-Alkoxyrest,

einer der Reste R2 und R4 einen 1-3C-Alkoxyrest und der andere ein Wasserstoffatom oder einen 1-3C-Alkylrest und

n die Zahlen 0 oder 1 darstellt,

sowie die Salze dieser Verbindungen.

2. Verbindungen der allgemeinen Formel I nach Anspruch 1, worin

R1 einen ganz oder überwiegend durch Fluor substituierten 1-3C-Alkylrest oder einen Chlordifluormethylrest,

R1' ein Wasserstoff- oder Halogenatom, Trifluormethyl, einen 1-3C-Alkylrest oder einen gegebenenfalls ganz oder überwiegend durch Fluor substituierten 1-3C-Alkoxyrest,

R3 einen 1-3C-Alkoxyrest,

einer der Reste R2 und R4 einen 1-3C-Alkoxyrest und der andere ein Wasserstoffatom oder einen 1-3C-Alkylrest und

n die Zahlen 0 oder 1 darstellt,

sowie die Salze dieser Verbindungen.

3. Verbindungen der allgemeinen Formel I nach Anspruch 1, worin

R1 und R1' gemeinsam und unter Einschluß des Sauerstoffatoms, an das R1 gebunden ist, einen gegebenenfalls ganz oder teilweise durch Fluor substituierten 1-2C-Alkylendioxyrest oder einen Chlortrifluorethylendioxyrest,

R3 einen 1-3C-Alkoxyrest,

einer der Reste R2 und R4 einen 1-3C-Alkoxyrest und der andere ein Wasserstoffatom oder einen 1-3C-Alkylrest und

n die Zahlen 0 oder 1 darstellt,

sowie die Salze dieser Verbindung.

4. Verbindungen der Formel I nach Anspruch 2, worin

R1 1,1,2,2-Tetrafluorethyl, Trifluormethyl, 2,2,2-Trifluorethyl oder Difluormethyl,

R1' ein Wasserstoffatom,

R3 Methoxy,

einer der Reste R2 oder R4 Methoxy und der andere ein Wasserstoffatom oder Methyl und

n die Zahlen 0 oder 1 darstellt,

und die Salze dieser Verbindungen.

5. Verbindungen der allgemeinen Formel I nach Anspruch 3, worin
R1 und R1′ gemeinsam und unter Einschluß des Sauerstoffatoms, an das R1 gebunden ist, einen Difluormethylendioxyrest oder einen Methylendioxyrest darstellen,
R3 Methoxy,
einer der Reste R2 oder R4 Methoxy und der andere ein Wasserstoffatom oder Methyl und
n die Zahlen 0 oder 1 darstellt,
und die Salze dieser Verbindungen.

6. Verbindungen der allgemeinen Formel I nach einem der Ansprüche 1 bis 5, worin n die Zahl 0 bedeutet, und ihre Säureadditionssalze.

7. Verbindungen der allgemeinen Formel I nach einem der Ansprüche 1 bis 5, worin n die Zahl 1 bedeutet, und ihre Salze mit Basen.

8. Verbindung ausgewählt aus der Gruppe bestehend aus
2-[(4,5-Dimethoxy-2-pyridyl)methylsulfinyl]-5-trifluormethoxy-1H-benzimidazol,
2-[(4,5-Dimethoxy-3-methyl-2-pyridyl)methylsulfinyl]-5-trifluormethoxy-1H-benzimidazol,
2-[(4,5-Dimethoxy-2-pyridyl)methylsulfinyl]-5-(1,1,2,2-tetrafluorethoxy)-1H-benzimidazol
2,2-Difluor-6-[(4,5-dimethoxy-2-pyridyl)methylthio]-5H-[1,3]-dioxolo[4,5-f]benzimidazol und
2,2-Difluor-6-[(4,5-dimethoxy-2-pyridyl)methylsulfinyl]-5H-[1,3]-dioxolo[4,5-f]benzimidazol
und ihren Salzen.

9. Verfahren zur Herstellung von Dialkoxypyridinen der allgemeinen Formel I nach Anspruch 1 und ihren Salzen, dadurch gekennzeichnet, daß man
a) Mercaptobenzimidazole der allgemeinen Formel II mit Picolinderivaten III,

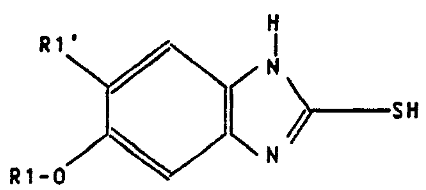
(II)

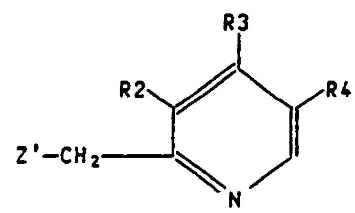
(III),

oder
b) Benzimidazole der allgemeinen Formel IV mit Mercaptopicolinen V

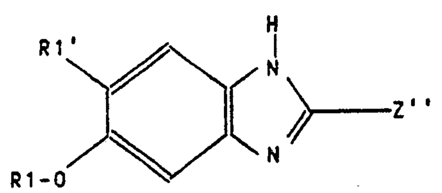
(IV)

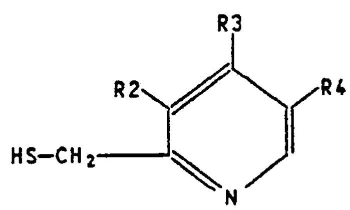
(V),

oder
c) o-Phenylendiamine der allgemeinen Formel VI mit Ameisensäurederivaten VII

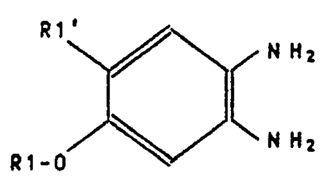
(VI)

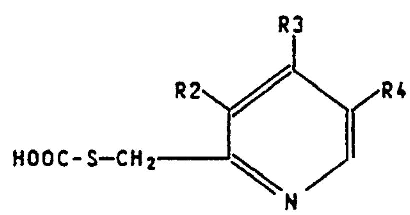
(VII),

umsetzt und gegebenenfalls anschließend die nach a), b) oder c) erhaltenen 2-Benzimidazolyl-2-pyridylmethylsulfide der allgemeinen Formel VIII

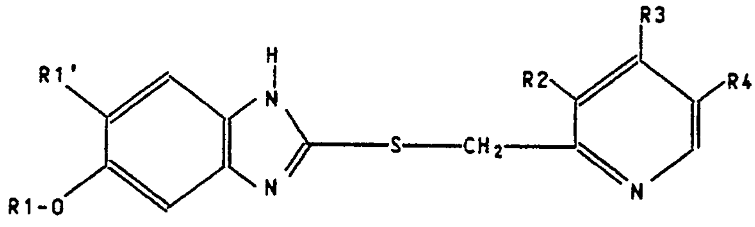

(VIII),

oxidiert und/oder in die Salze überführt,

oder daß man

d) Benzimidazole der allgemeinen Formel IX mit Pyridinderivaten X

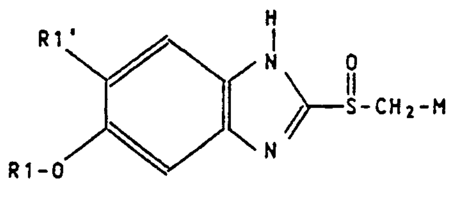

(IX)

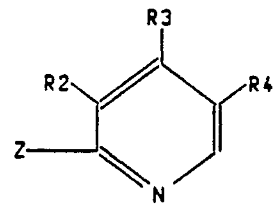

(X),

oder

e) Sulfinylderivate der allgemeinen Formel XI mit 2-Picolinderivaten XII

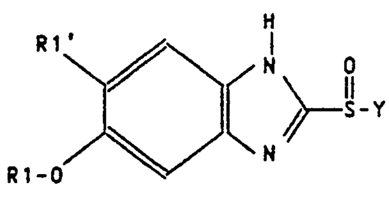

(XI)

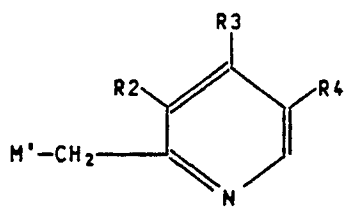

(XII),

umsetzt und gegebenenfalls anschließend in die Salze überführt,

wobei Y, Z, Z' und Z'' geeignete Abgangsgruppen darstellen,

M für ein Alkalimetallatom (Li, Na oder K) steht, M' für das Äquivalent eines Metallatoms steht und

R1, R1', R2, R3, R4 und n die in Anspruch 1 angegebenen Bedeutungen haben.

10. Arzneimittel enthaltend ein oder mehrere Dialkoxypyridine nach einem oder mehreren der Ansprüche 1 bis 8 und/oder ihre pharmakologisch verträglichen Salze.

11. Dialkoxypyridine nach einem der Ansprüche 1 bis 8 und ihre pharmakologisch verträglichen Salze zur Anwendung bei der Behandlung und/oder Prophylaxe von Krankheiten des Magens und/oder Darms und solcher Krankheiten, die auf einer erhöhten Magensäuresekretion beruhen.

12. Verwendung von Dialkoxypyridinen nach einem der Ansprüche 1 bis 8 und ihren pharmakologisch verträglichen Salzen zur Herstellung von Arzneimitteln für die Behandlung und/oder Prophylaxe von Krankheiten des Magens und/oder Darms und solchen Krankheiten, die auf einer erhöhten Magensäuresekretion beruhen.

13. 5-Difluormethoxy-2-[(3,4-dimethoxy-2-pyridyl)methylsulfinyl]-1H-benzimidazol und seine Salze.

**Patentansprüche** für den Vertragsstaat: AT

1. Verfahren zur Herstellung von Dialkoxypyridinen der allgemeinen Formel I

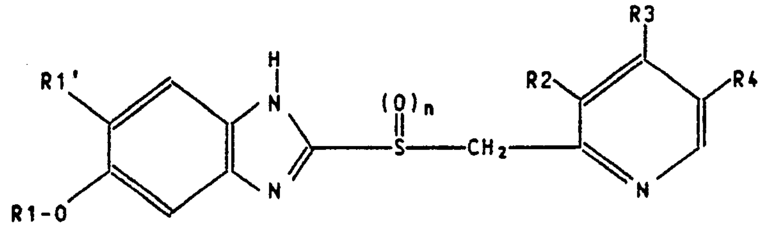

(I),

worin

R1 einen ganz oder überwiegend durch Fluor substituierten 1-3C-Alkylrest oder einen Chlordifluormethylrest und

R1' ein Wasserstoff- oder Halogenatom, Trifluormethyl, einen 1-3C-Alkylrest oder einen gegebenenfalls ganz oder überwiegend durch Fluor substituierten 1-3C-Alkoxyrest oder

R1 und R1' gemeinsam und unter Einschluß des Sauerstoffatoms, an das R1 gebunden ist, einen gegebenenfalls ganz oder teilweise durch Fluor substituierten 1-2C-Alkylendioxyrest oder einen Chlortrifluorethylendioxyrest darstellen,

R3 einen 1-3C-Alkoxyrest,

einer der Reste R2 und R4 einen 1-3C-Alkoxyrest und der andere ein Wasserstoffatom oder einen 1-3C-Alkylrest und

n die Zahlen 0 oder 1 darstellt,

und ihren Salzen, dadurch gekennzeichnet, daß man

a) Mercaptobenzimidazole der allgemeinen Formel II mit Picolinderivaten III

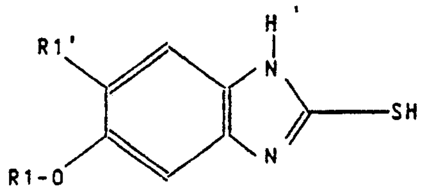

(II)

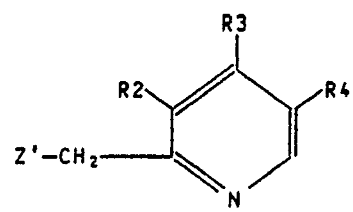

(III),

oder

b) Benzimidazole der allgemeinen Formel IV mit Mercaptopicolinen V

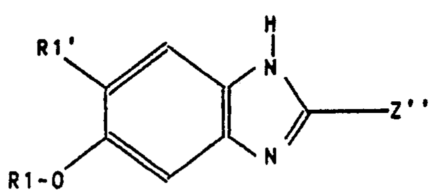

(IV)

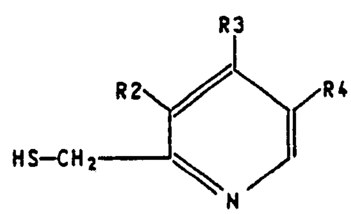

(V),

oder

c) o-Phenylendiamine der allgemeinen Formel VI mit Ameisensäurederivaten VII

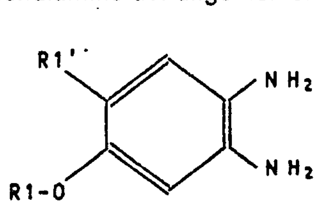

(VI)

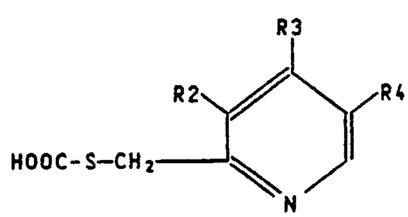

(VII),

umsetzt und gegebenenfalls anschließend die nach a), b) oder c) erhaltenen 2-Benzimidazolyl-2-pyridylmethylsulfide der allgemeinen Formel VIII

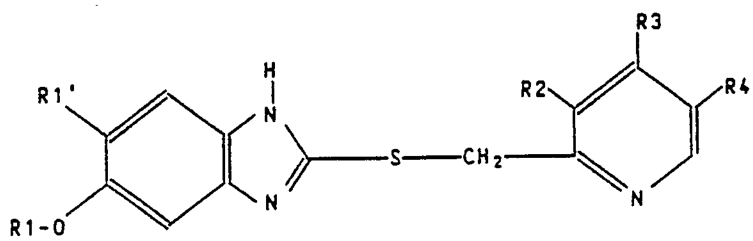

(VIII),

oxidiert und/oder in die Salze überführt,

oder daß man

d) Benzimidazole der allgemeinen Formel IX mit Pyridinderivaten X

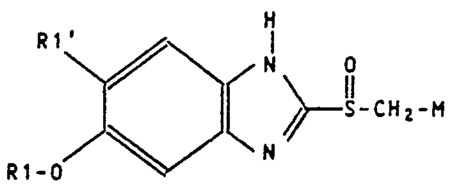

(IX)

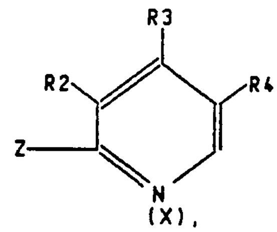

(X),

oder

e) Sulfinylderivate der allgemeinen Formel XI mit 2-Picolinderivaten XII

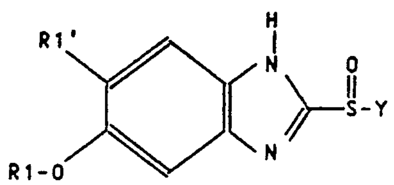

(XI)

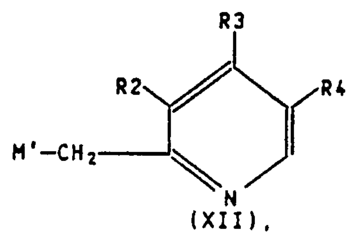

(XII),

umsetzt und gegebenenfalls anschließend in die Salze überführt, wobei

Y, Z, Z' und Z'' geeignete Abgangsgruppen darstellen,

M für ein Alkalimetallatom (Li, Na oder K) steht,

M' für das Äquivalent eines Metallatoms steht und R1, R1', R2, R3, R4 und n die oben angegebenen Bedeutungen haben.

2. Verfahren nach Anspruch 1, worin

R1 einen ganz oder überwiegend durch Fluor substituierten 1-3C-Alkylrest oder einen Chlordifluormethylrest,

R1' ein Wasserstoff- oder Halogenatom, Trifluormethyl, einen 1-3C-Alkylrest oder einen gegebenenfalls ganz oder überwiegend durch Fluor substituierten 1-3C-Alkoxyrest,

R3 einen 1-3C-Alkoxyrest,

einer der Reste R2 und R4 einen 1-3C-Alkoxyrest und der andere ein Wasserstoffatom oder einen 1-3C-Alkylrest und

n die Zahlen 0 oder 1 darstellt.

3. Verfahren nach Anspruch 1, worin

R1 und R1' gemeinsam und unter Einschluß des Sauerstoffatoms, an das R1 gebunden ist, einen gegebenenfalls ganz oder teilweise durch Fluor substituierten 1-2C-Alkylendioxyrest oder einen Chlortrifluorethylendioxyrest,

R3 einen 1-3C-Alkoxyrest,

einer der Reste R2 und R4 einen 1-3C-Alkoxyrest und der andere ein Wasserstoffatom oder einen 1-3C-Alkylrest und

n die Zahlen 0 oder 1 darstellt.

4. verfahren nach Anspruch 1, worin

R1 1,1,2,2-Tetrafluorethyl, Trifluormethyl, 2,2,2-Trifluorethyl oder Difluormethyl,

R1' ein Wasserstoffatom,

R3 Methoxy,

einer der Reste R2 oder R4 Methoxy und der andere ein Wasserstoffatom oder Methyl und

n die Zahlen 0 oder 1 darstellt.

5. Verfahren nach Anspruch 1, worin

R1 und R1' gemeinsam und unter Einschluß des Sauerstoffatoms, an das R1 gebunden ist, einen Difluormethylendioxyrest oder einen Methylendioxyrest darstellen,

R3 Methoxy,
einer der Reste R2 oder R4 Methoxy und der andere ein Wasserstoffatom oder Methyl und
n die Zahlen 0 oder 1 darstellt.

6. Verfahren zur Herstellung von Verbindungen der allgemeinen Formel I nach Anspruch 1, worin R1, R1', R2, R3 und R4 die in Anspruch 1 angegebenen Bedeutungen haben und n die Zahl 0 bedeutet, dadurch gekennzeichnet, daß man Mercaptobenzimidazole der allgemeinen Formel II mit Picolinderivaten III umsetzt und gegebenenfalls anschließend in die Säureadditionssalze überführt.

7. Verfahren zur Herstellung von Verbindungen der allgemeinen Formel I nach Anspruch 1, worin R1, R1', R2, R3 und R4 die in Anspruch 1 angegebenen Bedeutungen haben und n die Zahl 1 bedeutet, dadurch gekennzeichnet, daß man die 2-Benzimidazolyl-2-pyridylmethyl-sulfide der allgemeinen Formel VIII oxidiert und gegebenenfalls anschließend in die Salze mit Basen überführt.

8. Verfahren zur Herstellung von Arzneimitteln, dadurch gekennzeichnet, daß man eine Verbindung der allgemeinen Formel I nach Anspruch 1 oder ein pharmakologisch verträgliches Salz davon mit einem pharmazeutischen Hilfs- und/oder Trägerstoff vermischt.

9. Verfahren zur Herstellung von 5-Difluormethoxy-2-[(3,4-dimethoxy-2-pyridyl)methylsulfinyl]-1H-benzimidazol und seinen Salzen, dadurch gekennzeichnet, daß man 5-Difluormethoxy-2-[(3,4-dimethoxy-2-pyridyl)methylthio]-1H-benzimidazol oxidiert und gewünschtenfalls anschließend das erhaltene 5-Difluormethoxy-2-[(3,4-dimethoxy-2-pyridyl)methylsulfinyl]-1H-benzimidazol in ein Salz überführt.

10. Verfahren zur Herstellung von 5-Difluormethoxy-2-[(3,4-dimethoxy-2-pyridyl)methylthio]-1H-benzimidazol und seinen Salzen, dadurch gekennzeichnet, daß man 5-Difluormethoxy-2-mercapto-1H-benzimidazol mit 2-Chlormethyl-3,4-dimethoxypyridin oder seinem Salz umsetzt und gewünschtenfalls anschließend das erhaltene 5-Difluormethoxy-2-[(3,4-dimethoxy-2-pyridyl)methylthio]-1H-benzimidazol in ein Salz überführt oder ein erhaltenes Salz des 5-Difluormethoxy-2-[(3,4-dimethoxy-2-pyridyl)methylthio]-1H-benzimidazols in die freie Verbindung überführt.

**Claims** for the Contracting States: BE, CH, DE, FR, GB, IT, LI, LU, NL, SE

1. Dialkoxypyridines of the general formula I

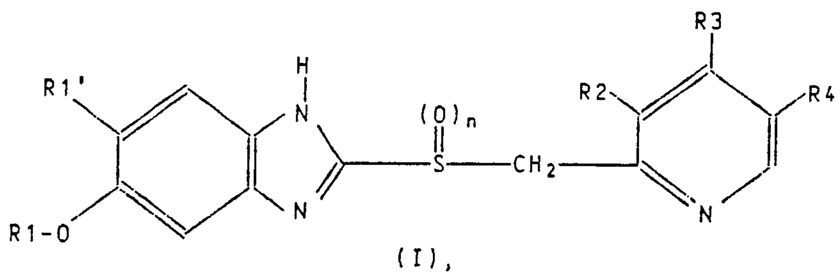

(I),

wherein
R1 represents a 1-3C-alkyl radical which is completely or predominantly substituted by fluorine, or a chlorodifluoromethyl radical and
R1' represents a hydrogen atom or a halogen atom, trifluoromethyl, a 1-3C-alkyl radical, or a 1-3C-alkoxy radical which is optionally completely or predominantly substituted by fluorine, or
R1 and R1' together, with inclusion of the oxygen atom to which R1 is bonded, represent a 1-2C-alkylenedioxy radical which is optionally completely or partly substituted by fluorine, or a chlorotrifluoroethylenedioxy radical,
R3 represents a 1-3C-alkoxy radical,
one of the radicals R2 and R4 represents a 1-3C-alkoxy radical and the other represents a hydrogen atom or a 1-3C-alkyl radical and
n represents the numbers 0 or 1,
and the salts of these compounds.

2. Compounds of the general formula I according to claim 1, wherein
R1 represents a 1-3C-alkyl radical which is completely or predominantly substituted by fluorine, or a chlorodifluoromethyl radical,
R1' represents a hydrogen atom or a halogen atom, trifluoromethyl, a 1-3C-alkyl radical, or a 1-3C-alkoxy radical which is optionally completely or predominantly substituted by fluorine,
R3 represents a 1-3C-alkoxy radical,
one of the radical R2 and R4 represents a 1-3C-alkoxy radical and the other represents a hydrogen atom or a 1-3C-alkyl radical and
n represents the numbers 0 or 1,
and the salts of these compounds.

3. Compounds of the general formula I according to claim 1, wherein
R1 and R1' together and with inclusion of the oxygen atom to which R1 is bonded, represent a 1-2C-

alkylenedioxy radical which is optionally completely or partly substituted by fluorine, or a chlorotrifluoroethylenedioxy radical,

R3 represents a 1-3C-alkoxy radical,

one of the radicals R2 and R4 represents a 1-3C-alkoxy radical and the other represents a hydrogen atom or a 1-3C-alkyl radical and

n represents the numbers 0 or 1,

and the salts of these compounds.

4. Compounds of the general formula I according to claim 2, wherein

R1 represents 1,1,2,2-tetrafluoroethyl, trifluoromethyl, 2,2,2-trifluoroethyl or difluoromethyl,

R1' represents a hydrogen atom,

R3 represents methoxy,

one of the radicals R2 and R4 represents methoxy and the other represents a hydrogen atom or methyl and

n represents the numbers 0 or 1,

and the salts of these compounds.

5. Compounds of the general formula I according to claim 3, wherein

R1 and R1' together, with inclusion of the oxygen atom to which R1 is bonded, represent a difluoromethylenedioxy radical or a methylenedioxy radical,

R3 represents methoxy,

one of the radicals R2 and R4 represents methoxy and the other represents a hydrogen atom or methyl and

n represents the numbers 0 or 1,

and the salts of these compounds.

6. Compounds of the general formula I according to one of claims 1 to 5, wherein n denotes the number 0, and their acid addition salts.

7. Compounds of the general formula I according to one of claims 1 to 5, wherein n denotes the number 1, and their salts with bases.

8. A compound chosen from the group consisting of

2-[(4,5-dimethoxy-2-pyridyl)methylsulfinyl]-5-trifluoromethoxy-1H-benzimidazole,

2-[(4,5-dimethoxy-3-methyl-2-pyridyl)methylsulfinyl]-5-trifluoromethoxy-1H-benzimidazole,

2-[(4,5-dimethoxy-2-pyridyl)methylsulfinyl]-5-(1,1,2,2,-tetrafluoroethoxy)-1H-benzimidazole,

2,2-difluoro-6-[(4,5-dimethoxy-2-pyridyl)methylthio]-5H-[1,3]-dioxolo[4,5-f]benzimidazole and

2,2-difluoro-6-[(4,5-dimethoxy-2-pyridyl)methylsulfinyl]-5H-[1,3]-dioxolo[4,5-f]benzimidazole and their salts.

9. Process for the preparation of dialkoxypyridines of the general formula I according to claim 1 and their salts, characterized in that:

a) mercaptobenzimidazoles of the general formula II are reacted with picoline derivatives III,

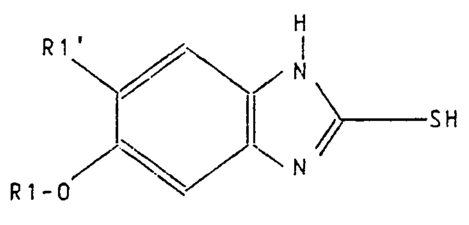

(II)

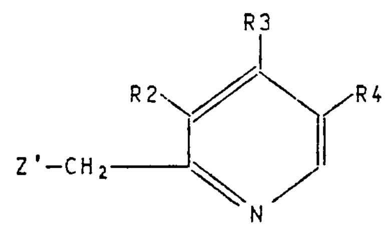

(III),

or

b) benzimidazoles of the general formula IV are reacted with mercaptopicolines V,

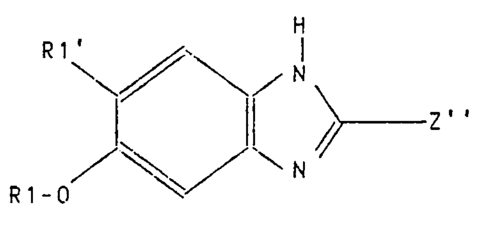

(IV)

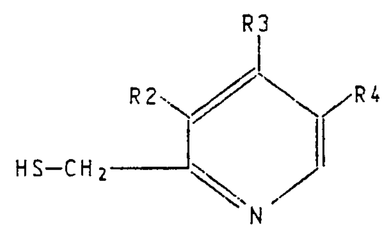

(V),

or

c) o-phenylenediamines of the general formula VI are reacted with formic acid derivatives VII

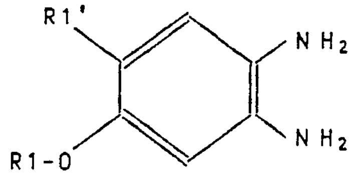

(VI)

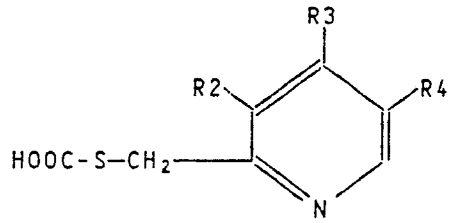

(VII),

and the 2-benzimidazolyl 2-pyridylmethylsulfides of the general formula VIII obtained according to a), b) or c)

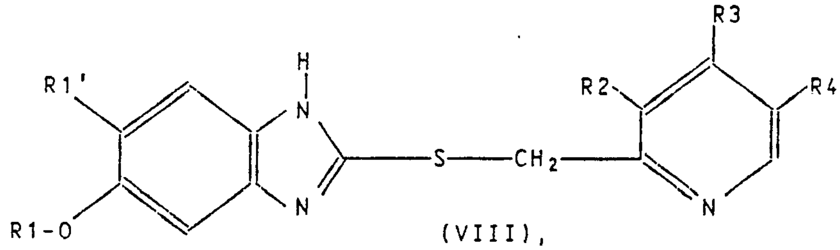

(VIII),

are then optionally oxidized and/or converted into the salts, or

d) benzimidazoles of the general formula IX are reacted with pyridine derivatives X

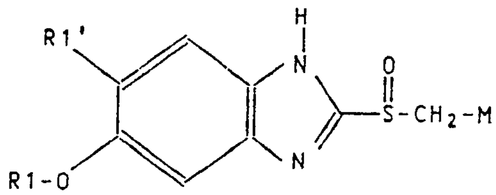

(IX)

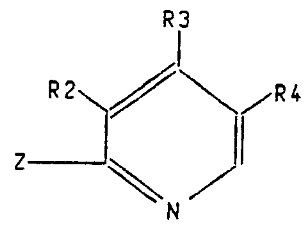

(X),

e) sulfinyl derivatives of the general formula XI are reacted with 2-picoline derivatives XII

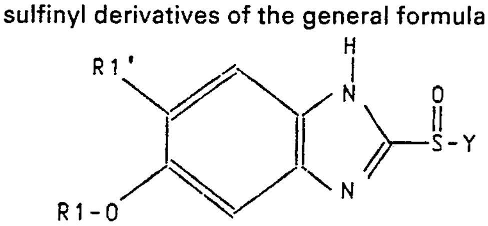

(XI)

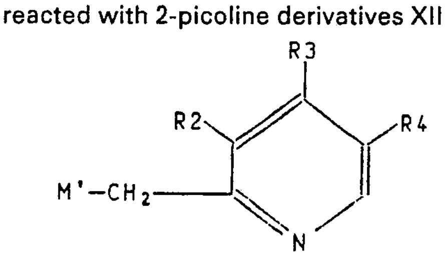

(XII),

and are then optionally subsequently converted into the salts,

Y, Z, Z' and Z'' representing suitable leaving groups,

M representing an alkali metal atom (Li, Na or K),

M' representing the equivalent of a metal atom and

R1, R1', R2, R3, R4 and n having the meanings given in claim 1.

10. Medicaments containing one or more dialkoxypyridines according to one or more of the claims 1 to 8 and/or their pharmacologically acceptable salts.

11. Dialkoxypyridines according to one of claims 1 to 8 and their pharmacologically acceptable salts for use in the treatment and/or prophylaxis of diseases of the stomach and/or intestine and diseases based on increased secretion of gastric acid.

12. Use of dialkoxypyridines according to one of claim 1 to 8 and their pharmacologically acceptable salts for the manufacture of medicaments for the treatment and/or prophylaxis of diseases of the stomach and/or intestine and diseases based on increased secretion of gastric acid.

13. 5-Difluoromethoxy-2-[(3,4-dimethoxy-2-pyridyl)methylsulfinyl]-1H-benzimidazole and its salts.

**Claims** for the Contracting State: AT

1. Process for the preparation of dialkoxypyridines of the general formula

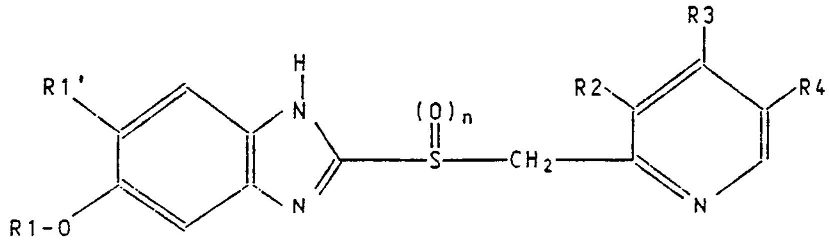

(I),

wherein

R1 represents a 1-3C-alkyl radical which is completely or predominantly substituted by fluorine, or a chlorodifluoromethyl radical and

R1′ represents a hydrogen atom or a halogen atom, trifluoromethyl, a 1-3C-alkyl radical, or a 1-3C-alkoxy radical which is optionally completely or predominantly substituted by fluorine, or

R1 and R1′ together, with inclusion of the oxygen atom to which R1 is bonded, represent a 1-2C-alkylenedioxy radical which is optionally completely or partly substituted by fluorine, or a chlorotrifluoroethylenedioxy radical,

R3 represents a 1-3C-alkoxy radical,

one of the radicals R2 and R4 represents a 1-3C-alkoxy radical and the other represents a hydrogen atom or a 1-3C-alkyl radical and

n represents the numbers 0 or 1,

and their salts, characterized in that

a) mercaptobenzimidazoles of the general formula II are reacted with picoline derivatives III,

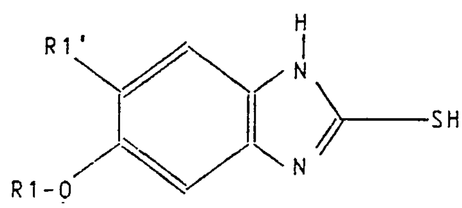

(II)

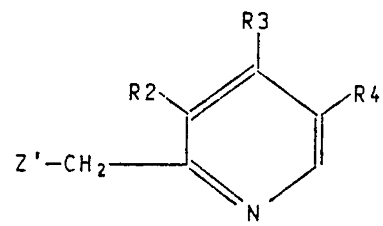

(III),

b) benzimidazoles of the general formula IV are reacted with mercaptopicolines V,

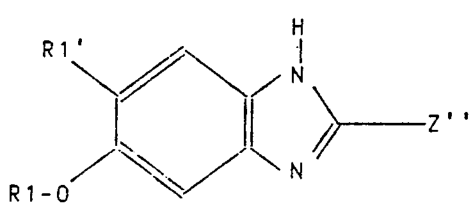

(IV)

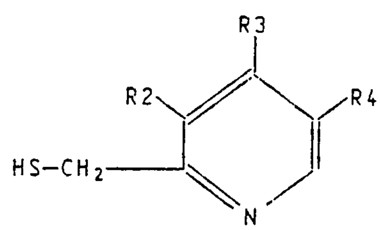

(V),

or

c) o-phenylenediamines of the general formula VI are reacted with formic acid derivatives VII

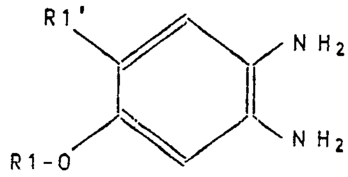

(VI)

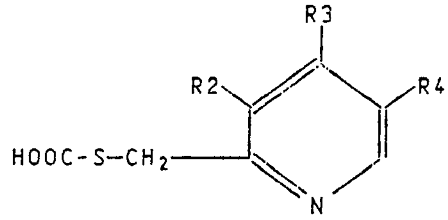

(VII),

and the 2-benzimidazolyl 2-pyridylmethylsulfides of the general formula VIII obtained according to a), b) or c)

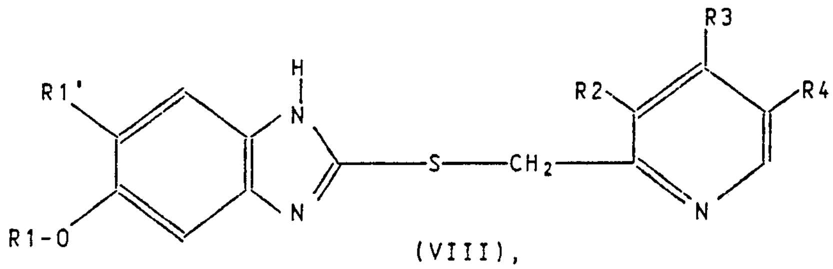

(VIII),

are then optionally oxidized and/or converted into the salts,

or

d) benzimidazoles of the general formula IX are reacted with pyridine derivatives X

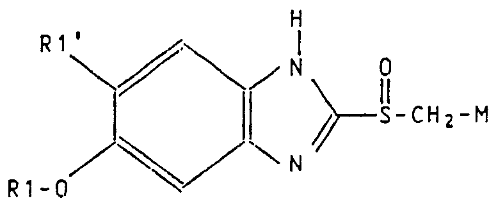

(IX)

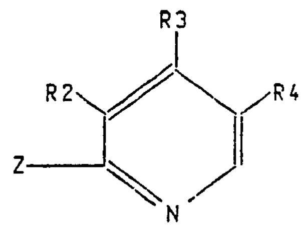

(X),

or

e) sulfinyl derivatives of the general formula XI are reacted with 2-picoline derivatives XII

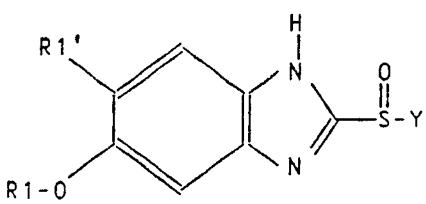

(XI)

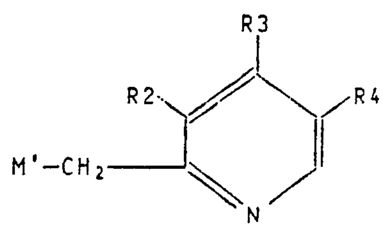

(XII),

and are then optionally subsequently converted into the salts,

Y, Z, Z' and Z'' representing suitable leaving groups,

M representing an alkali metal atom (Li, Na or K),

M' representing the equivalent of a metal atom and

R1, R1', R2, R3, R4 and n having the meanings given above.

2. Process according to claim 1, wherein

R1 represents a 1-3C-alkyl radical which is completely or predominantly substituted by fluorine, or a chlorodifluoromethyl radical,

R1' represents a hydrogen atom or a halogen atom, trifluoromethyl, a 1-3C-alkyl radical, or a 1-3C-alkoxy radical which is optionally completely or predominantly substituted by fluorine,

R3 represents a 1-3C-alkoxy radical,

one of the radical R2 and R4 represents a 1-3C-alkoxy radical and the other represents a hydrogen atom or a 1-3C-alkyl radical and

n represents the numbers 0 or 1.

3. Process according to claim 1, wherein

R1 and R1' together and with inclusion of the oxygen atom to which R1 is bonded, represent a 1-2C-alkylenedioxy radical which is optionally completely or partly substituted by fluorine, or a chlorotrifluoroethylenedioxy radical,

R3 represents a 1-3C-alkoxy radical,

one of the radicals R2 and R4 represents a 1-3C-alkoxy radical and the other represents a hydrogen atom or a 1-3C-alkyl radical and

n represents the numbers 0 or 1.

4. Process according to claim 1, wherein

R1 represents 1,1,2,2-tetrafluoroethyl, trifluoromethyl, 2,2,2-trifluoroethyl or difluoromethyl,

R1' represents a hydrogen atom,

R3 represents methoxy,

one of the radicals R2 and R4 represents methoxy and the other represents a hydrogen atom or methyl and

n represents the numbers 0 or 1.

5. Process according to claim 1, wherein

R1 and R1' together, with inclusion of the oxygen atom to which R1 is bonded, represent a difluoromethylenedioxy radical or a methylenedioxy radical,

R3 represents methoxy,

one of the radicals R2 and R4 represents methoxy and the other represents a hydrogen atom or methyl and

n represents the numbers 0 or 1.

6. Process for the preparation of compounds of the general formula I according to claim 1, wherein R1, R1', R2, R3 and R4 have the meanings given in claim 1 and n represents the number 0, characterized in that mercaptobenzimidazoles of the general formula II are reacted with picoline derivatives III and are then optionally subsequently converted into the acid addition salts.

7. Process for the preparation of compounds of the general formula I according to claim 1, wherein R1, R1', R2, R3 and R4 have the meanings given in claim 1 and n represents the number 1, characterized in that the 2-benzimidazolyl 2-pyridylmethylsulfides of the general formula VIII are oxidized and are then optionally subsequently converted into the salts with bases.

8. Process for the preparation of medicaments, characterized in that a compound of the general formula I according to claim 1 or a pharmacologically acceptable salt thereof is mixed with a pharmaceutical auxiliary and/or carrier.

9. Process for the preparation of 5-difluoromethoxy-2-[(3,4-dimethoxy-2-pyridyl)methylsulfinyl]-1H-benzimidazole and its salts, characterized in that 5-difluoromethoxy-2-[(3,4-dimethoxy-2-pyridyl)methylthio]-1H-benzimidazole is oxidized and, if desired, the resulting 5-difluoromethoxy-2-[(3,4-dimethoxy-2-pyridyl)methylsulfinyl]-1H-benzimidazole is subsequently converted into a salt.

10. Process for the preparation of 5-difluoromethoxy-2-[(3,4-dimethoxy-2-pyridyl)methylthio]-1H-benzimidazole and its salts, characterized in that 5-difluoromethoxy-2-mercapto-1H-benzimidazole is reacted with 2-chloromethyl-3,4-dimethoxypyridine or its salt, and, if desired, the resulting 5-difluoromethoxy-2-[(3,4-dimethoxy-2-pyridyl)methylthio]-1H-benzimidazole is subsequently converted into a salt or a resulting salt of the 5-difluoromethoxy-2-[(3,4-dimethoxy-2-pyridyl)methylthio]-1H-benzimidazole is subsequently converted into the free compound.

**Revendications** pour les Etats Contractants: BE, CH, DE, FR, GB, IT, LI, LU, NL SE

1. Dialkoxypyridines répondant à la formule générale:

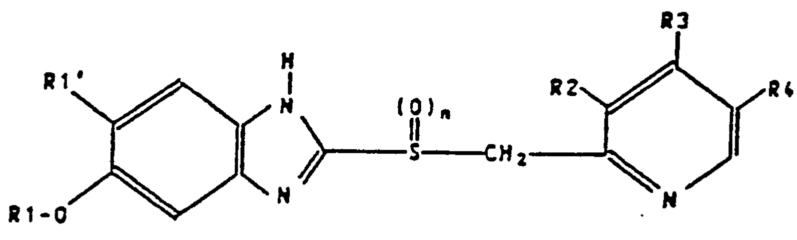

(I),

dans laquelle

R1 représente un reste alkyle en $C_1$ à $C_3$, substitué en totalité ou en majeure partie par du fluor, ou un reste chlorodifluorométhyle, et

R1' représente un atome d'hydrogène ou d'halogène, un reste trifluorométhyle, un reste alkyle en $C_1$ à $C_3$ ou un reste alkoxy en $C_1$ à $C_3$ éventuellement substitué en totalité ou en majeure partie par du fluor, ou bien R1 et R1' forment avec l'atome d'oxygène auquel R1 est lié un reste alkylène-dioxy en $C_1$ ou $C_2$, éventuellement substitué en totalité ou en partie par du fluor, ou un reste chlorotrifluoroéthylène-dioxy,

R3 représente un reste alkoxy en $C_1$ à $C_3$,

l'un des restes R2 et R4 représente un reste alkoxy en $C_1$ à $C_3$ et l'autre un atome d'hydrogène ou un reste alkyle en $C_1$ à $C_3$, et

n représente les nombres 0 ou 1,

ainsi que les sels de ces composés.

2. Composés répondant à la formule générale I selon la revendication 1, où

R1 représente un reste alkyle en $C_1$ à $C_3$ substitué en totalité ou en majeure partie par du fluor, ou un reste chlorodifluorométhyle,

R1′ représente un atome d'hydrogène ou d'halogène, un reste trifluorométhyle, un reste alkyle en $C_1$ à $C_3$ ou un reste alkoxy en $C_1$ à $C_3$ éventuellement substitué en totalité ou en majeure partie par du fluor,

R3 représente un reste alkoxy en $C_1$ à $C_3$,

l'un des restes R2 et R4 représente un reste alkoxy en $C_1$ à $C_3$ et l'autre un atome d'hydrogène ou un reste alkyle en $C_1$ à $C_3$, et

n représente les nombres 0 ou 1,

ainsi que les sels de ces composés.

3. Composés répondant à la formule générale I selon la revendication 1, où

R1 et R1′ forment avec l'atome d'oxygène auquel le reste R1 est lié, un reste alkylène-dioxy en $C_1$ ou $C_2$, éventuellement substitué en totalité ou en partie par du fluor, ou un reste chlorotrifluoroéthylène-dioxy,

R3 représente un reste alkoxy en $C_1$ à $C_3$,

l'un des restes R2 et R4 représente un reste alkoxy en $C_1$ à $C_3$ et l'autre un atome d'hydrogène ou un reste alkyle en $C_1$ à $C_3$, et

n représente les nombres 0 ou 1,

ainsi que les sels de ces composés.

4. Composés répondant à la formule générale I selon la revendication 2, où

R1 représente un reste 1,1,2,2-tétrafluoroéthyle, trifluorométhyle, 2,2,2-trifluoroéthyle ou difluorométhyle,

R1′ représente un atome d'hydrogène,

R3 un reste méthoxy,

l'un des restes R2 ou R4 un reste méthoxy et l'autre un atome d'hydrogène ou un groupe méthyle, et

n représente les nombres 0 ou 1,

et les sels de ces composés.

5. Composés répondant à la formule générale I selon la revendication 3, où

R1 et R1′ forment avec l'atome d'oxygène auquel R1 est lié, un reste difluorométhylène-dioxy ou un reste méthylène-dioxy,

R3 un reste méthoxy,

l'un des restes R2 ou R4 un reste méthoxy et l'autre un atome d'hydrogène ou un groupe méthyle, et

n les nombres 0 ou 1, et les sels de ces composés.

6. Composés répondant à la formule générale I selon l'une quelconque des revendications 1 à 5, où n représente le nombre 0, et leurs sels d'addition acides.

7. Composés répondant à la formule générale I selon l'une quelconque des revendications 1 à 5, où n représente le nombre 1, et leurs sels avec des bases.

8. Composé choisi dans le groupe comprenant les

2-[(4,5-diméthoxy-2-pyridyl)méthylsulfinyl]-5-trifluorométhoxy-1H-benzimidazole,

2-[(4,5-diméthoxy-3-méthyl-2-pyridyl)méthylsulfinyl]-5-trifluorométhoxy-1H-benzimidazole,

2-[(4,5-diméthoxy-2-pyridyl)méthylsulfinyl]-5-(1,1,2,2-tétrafluoroéthoxy)-1H-benzimidazole,

2,2-difluoro-6-[(4,5-diméthoxy-2-pyridyl)méthylthio]-5H-[1,3]-dioxolo-[4,5-f]benzimidazole, et

2,2-difluoro-6-[(4,5-diméthoxy-2-pyridyl)méthylsulfinyl]-5H-[1,3]-dioxolo-[4,,5-f]benzimidazole.

et leurs sels.

9. Procédé de préparation de dialkoxypyridines répondant à la formule générale I selon la revendication 1 et leurs sels, caractérisé en ce que l'on fait réagir a) des mercaptobenzimidazoles répondant à la formule générale II avec des dérivés de picoline III:

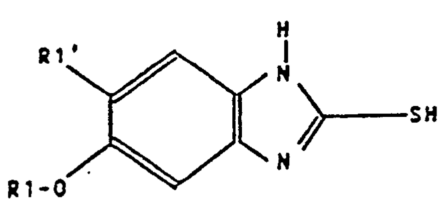

(II)

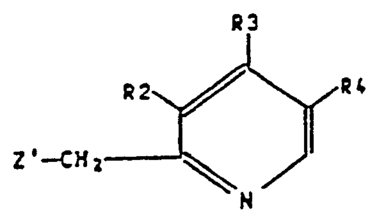

(III).

b) des benzimidazoles répondant à la formule générale IV avec des mercaptopicolines V

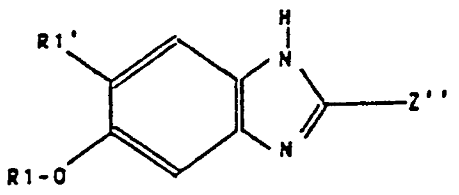

(IV)

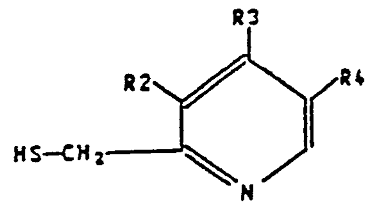

(V).

c) des o-phénylènediamines répondant à la formule générale VI avec des dérivés de l'acide formique VII

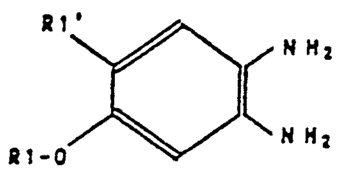

(VI)

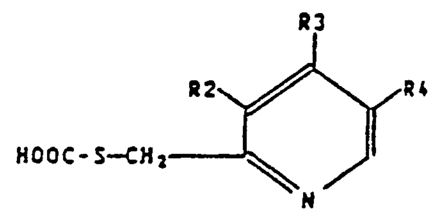

(VII),

et éventuellement, subséquemment, on oxyde les sulfures de 2-benzimidazolyl-2-pyridylméthyle répondant à la formule générale VIII, obtenus en a), b) ou c):

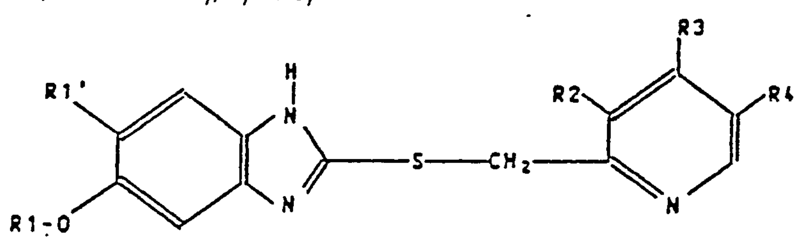

(VIII),

et/ou on les transforme en sels,

ou bien l'on fait réagir

d) des benzimidazoles répondant à la formule générale IX avec des dérivés de la pyridine X

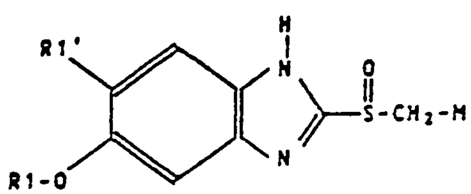

(IX)

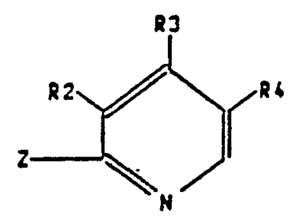

(X),

ou

e) des dérivés sulfinyliques répondant à la formule générale XI avec des dérivés de 2-picoline XII

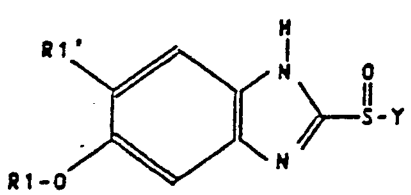

(XI)

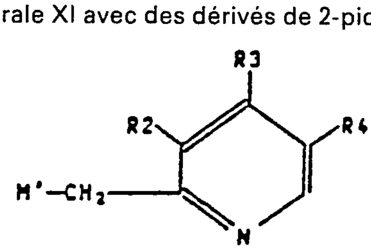

(XII),

et éventuellement l'on effectue subséquemment une transformation en sels,

Y, Z, Z' et Z'' représentant des groupes scindables appropriés,

M représentant un atome de métal alcalin (Li, Na ou K), et

M' représentant l'équivalent d'un atome métallique,

et R1, R1', R2, R3, R4 et n ayant les significations indiquées dans la revendication 1.

10. Médicament comprenant une ou plusieurs dialkoxypyridines selon l'une quelconque des revendications 1 à 8 et/ou leurs sels pharmacologiquement compatibles.

11. Application des dialkoxypyridines selon l'une quelconque des revendications 1 à 8 et leurs sels pharmacologiquement compatibles, à appliquer au traitement et/ou à la prophylaxie de maladies de l'estomac et/ou de l'intestin, et de maladies reposant sur une sécrétion accrue en acides stomacaux.

12. Application de dialkoxypyridines selon l'une quelconque des revendications 1 à 8 et de leurs sels pharmacologiquement compatibles à la préparation de médicaments destinés au traitement et/ou à la prophylaxie de maladies de l'estomac et/ou de l'intestin et de maladies reposant sur une sécrétion accrue en acides stomacaux.

13. 5-Difluorométhoxy-2-[(3,4-diméthoxy-2-pyridyl)méthylsulfinyl]-1H-benzimidazole, et ses sels.

**Revendications** pour l'Etat Contractant: AT

1. Procédé de préparation de dialkoxypyridines répondant à la formule générale I:

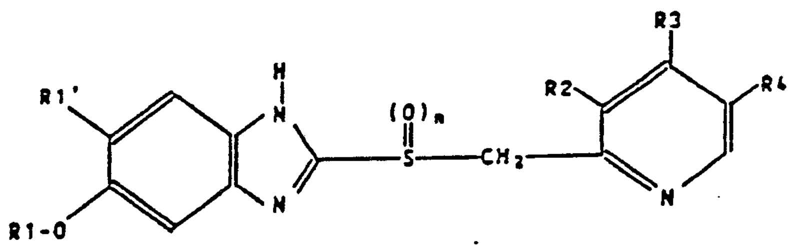

(I),

dans laquelle

R1 représente un reste alkyle en $C_1$ à $C_3$ substitué en totalité ou en majeure partie par du fluor, ou un reste chlorodifluorométhyle, et

R1' représente un atome d'hydrogène ou d'halogène, un reste trifluorométhyle, un reste alkyle en $C_1$ à $C_3$ ou un reste alkoxy éventuellement substitué en totalité ou en majeure partie par du fluor, ou bien

R1 et R1' forment avec l'atome d'oxygène auquel R1 est lié un reste alkylène-dioxy en $C_1$ ou $C_2$ éventuellement substitué en totalité ou en partie par du fluor, ou un reste chlorotrifluoro-éthylène-dioxy,

R3 représente un reste alkoxy en $C_1$ à $C_3$, l'un des restes R2 et R4 représente un reste alkoxy en $C_1$ à $C_3$ et l'autre un atome d'hydrogène ou un reste alkyle en $C_1$ à $C_3$, et

n représente les nombres 0 ou 1,

et leurs sels, caractérisé en ce que l'on fait réagir

a) des mercaptobenzimidazoles répondant à la formule générale II avec des dérivés de picoline III

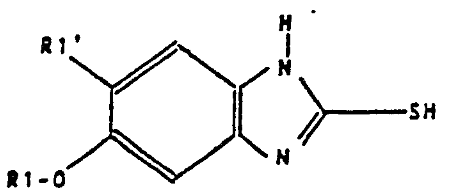

(II)

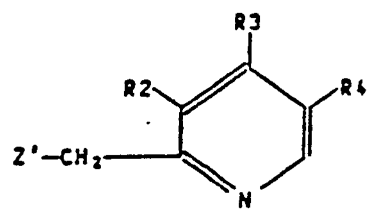

(III),

ou

b) des benzimidazoles répondant à la formule générale IV avec des mercaptopicolines V

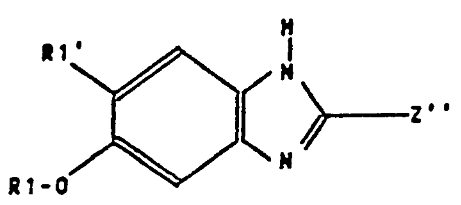

(IV)

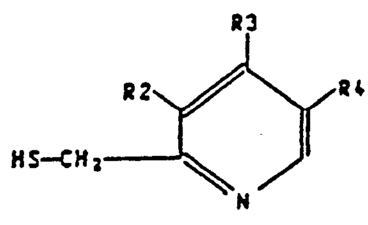

(V),

ou

c) des o-phénylènediamines répondant à la formule générale VI avec des dérivés de l'acide formique VII

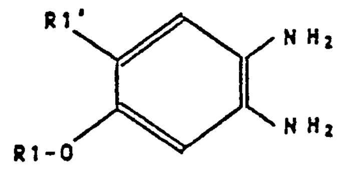

(VI)

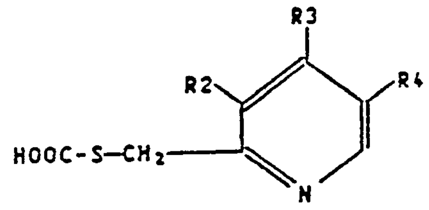

(VII),

et éventuellement, subséquemment, l'on oxyde les sulfures de 2-benzimidazolyl-2-pyridylméthyle répondant à la formule générale VIII, obtenus en a), b) ou c)

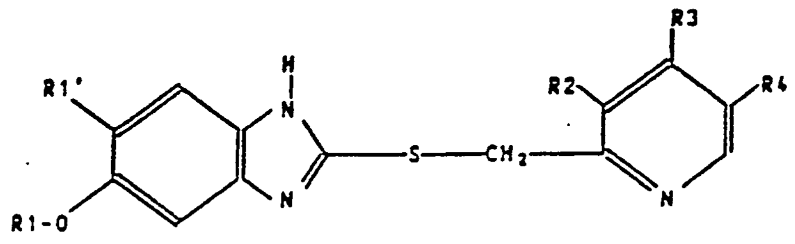

(VIII),

et/ou on les transforme en sels, ou bien l'on fait réagir

d) des benzimidazoles répondant à la formule générale IX avec des dérivés de la pyridine X

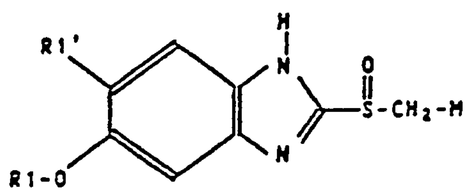

(IX)

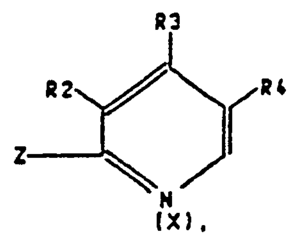

(X),

e) des dérivés sulfinyliques répondant à la formule générale XI avec des dérivés de 2-picoline XII

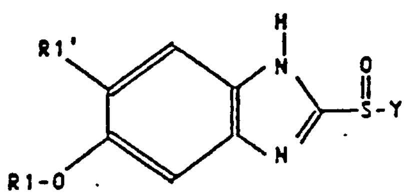

(XI)

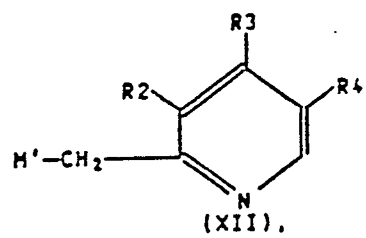

(XII),

et éventuellement, subséquemment, l'on effectue la transformation en sels,

Y, Z, Z' et Z'' représentant des croupes scindables appropriés,

M représentant un atome de métal alcalin (Li, Na ou K),

M' représentant l'équivalent d'un atome métallique, et

R1, R1', R2, R3, R4 et n ayant les significations indiquées précédemment.

2. Procédé selon la revendication 1, où

R1 représente un reste alkyle en $C_1$ à $C_3$ substitué en totalité ou en majeure partie par du fluor, ou un reste chlorodifluorométhyle,

R1' représente un atome d'hydrogène ou d'halogène, un reste trifluorométhyle, un reste alkyle en $C_1$ à $C_3$ ou un reste alkoxy en $C_1$ à $C_3$ éventuellement substitué en totalité ou en majeure partie par du fluor,

R3 représente un reste alkoxy en $C_1$ à $C_3$,

l'un des restes R2 et R4 représente un reste alkoxy en $C_1$ à $C_3$ et l'autre un atome d'hydrogène ou un reste alkyle en $C_1$ à $C_3$, et

n représente les nombres 0 ou 1.

3. Procédé selon la revendication 1, où

R1 et R1' forment avec l'atome d'oxygène auquel R1 est lié un reste alkylène-dioxy en $C_1$ ou $C_2$, éventuellement substitué en totalité ou en partie par du fluor, ou un reste chlorotrifluoro-éthylène-dioxy,

R3 représente un reste alkoxy en $C_1$ à $C_3$,

l'un des restes R2 et R4 représente un reste alkoxy en $C_1$ à $C_3$ et l'autre un atome d'hydrogène ou un reste alkyle en $C_1$ à $C_3$, et

n représente les nombres 0 ou 1.

4. Procédé selon la revendication 1, où

R1 représente un reste 1,1,2,2-tétrafluoroéthyle, trifluorométhyle, 2,2,2-trifluoroéthyle ou difluorométhyle, R1' représente un atome d'hydrogène,

R3 représente un reste méthoxy,

l'un des restes R2 ou R4 représente un reste méthoxy et l'autre un atome d'hydrogène ou un reste méthyle, et

n représente les nombres 0 ou 1.

5. Procédé selon la revendication 1, où

R1 et R1' forment avec l'atome d'oxygène auquel R1 est lié un reste difluorométhylène-dioxy ou un reste méthylène-dioxy,

R3 un reste méthoxy,

l'un des restes R2 ou R4 un reste méthoxy et l'autre un atome d'hydrogène ou un reste méthyle et

n les nombres 0 ou 1.

6. Procédé de préparation de composés répondant à la formule générale I selon la revendication 1, où R1, R1', R2, R3 et R4 ont les significations indiquées dans la revendication 1 et n représente le nombre 0, caractérisé en ce qu'on fait réagir des mercapto-benzimidazoles répondant à la formule générale II avec des dérivés de picoline III et éventuellement subséquemment, on effectue la transformation en sels d'addition acides.

7. Procédé de préparation de composés répondant à la formule générale I selon la revendication 1, où R1, R1', R2, R3 et R4 ont les significations indiquées dans la revendication 1 et n représente le nombre 1, caractérisé en ce qu'on oxyde les sulfures de 2-benzimidazolyl-2-pyridylméthyle répondant à la formule générale VIII et on les transforme éventuellement, subséquemment, en sels avec des bases.

8. Procédé de préparation de médicaments, caractérisé en ce qu'on mélange un composé répondant à la formule générale I selon la revendication 1 ou un de ses sels pharmacologiquement compatible avec un adjuvant pharmaceutique et/ou un support pharmaceutique.

9. Procédé de préparation de 5-difluorométhoxy-2-[(3,4-diméthoxy-2-pyridyl)méthylsulfinyl]-1H-benzimidazole et de ses sels, caractérisé en ce qu'on oxyde le 5-difluorométhoxy-2-[(3,4-diméthoxy-2-pyridyl)méthylthio]-1H-benzimidazole et subséquemment, si on le souhaite, on transforme le 5-difluorométhoxy-2-[(3,4-diméthoxy-2-pyridyl)méthylsulfinyl]-1H-benzimidazole ainsi obtenu en un sel.

10. Procédé de préparation de 5-difluorométhoxy-2-[(3,4-diméthoxy-2-pyridyl)méthylthio]-1H-benzimidazole et de ses sels, caractérisé en ce qu'on fait réagir le 5-difluorométhoxy-2-mercapto-1H-benzimidazole avec la 2-chlorométhyl-3,4-diméthoxypyridine ou son sel et subséquemment, si on le souhaite, on transforme le 5-difluorométhoxy-2-[(3,4-diméthoxy-2-pyridyl)méthylthio]-1H-benzimidazole ainsi obtenu en un sel, ou bien l'on transforme le sel du 5-difluorométhoxy-2-[(3,4-diméthoxy-2-pyridyl)méthylthio]-1H-benzimidazole obtenu en son composé libre.